# EUROPEAN PATENT SPECIFICATION

(11) **EP 1 984 121 B1**
(45) Date of publication and mention of the grant of the patent: **08.05.2019**
(21) Application number: 07718288.9
(22) Date of filing: 16.01.2007
(51) Int. Cl.: B05D 3/00, A61F 2/00, A61L 27/34, A61L 29/08, A61L 31/10, A61L 15/46, A61L 15/48, A61L 27/54, A61L 29/16, A61L 31/16, C08G 18/08, C08G 18/10

(54) **NON-LEACHING SURFACE-ACTIVE FILM COMPOSITIONS FOR MICROBIAL ADHESION PREVENTION**
NICHT AUSLAUGENDE OBERFLÄCHENAKTIVE FOLIENZUSAMMENSETZUNG ZUR VERHINDERUNG VON MIKROBIELLER ADHÄSION
COMPOSITIONS DE FILM TENSIOACTIF NON SUJET À LIXIVIATION POUR EMPÊCHER UNE ADHÉSION MICROBIENNE

(30) Priority: 18.01.2006 US 334049
(43) Date of publication of application: 29.10.2008
(73) Proprietor: HYDROMER, INC., Branchburg, NJ 08876 (US)
(72) Inventor: QU, Xin, East Brunswick, NJ 08816 (US); GRUENING, Rainer, Basking Ridge, NJ 07920 (US); MERRITT, Karen, Hillsborough, NJ 08844 (US); CHEN, Paul, N., Canandaigua, NY 14424 (US); FALEVICH, Vitaly, York, Pennsylvania 17402 (US)
(74) Representative: V.O.
(86) International application number: PCT/US2007/001026
(87) International publication number: WO 2007/084452

(56) References cited:
- EP-A1- 1 329 470
- WO-A1-99/33344
- WO-A2-02/10244
- US-A- 5 084 096
- US-A- 5 998 200
- US-A1- 2002 177 828
- US-A1- 2004 109 853
- US-A1- 2005 042 240
- US-A1- 2005 095 351
- US-A1- 2005 096 249
- US-A1- 2005 191 270
- US-A1- 2005 249 695
- US-A1- 2005 249 791
- US-A1- 2005 271 743
- US-B1- 6 203 317
- US-B1- 6 716 895

## Description

### BACKGROUND OF INVENTION

### Field of Invention

The present invention relates to surface-active, non-leaching antimicrobial coating compositions. The curable antimicrobial coating compositions may be applied to a surface to provide the surface with non-leaching anti-microbial properties. The compositions of the present invention form durable coatings with long-lasting anti-microbial efficacy without formation of a zone of inhibition. The compositions according to the present invention are also directed to durable non-leaching coatings which exhibit a reduced tendency for blood coagulation.

### Background

Microorganisms can grow and multiply in the presence of water and suitable temperature conditions with enormous speed. It is estimated that under favorable temperature and moisture conditions a microbial, e.g., bacterial, population can double every 20 minutes. Protection from dangerous levels of microbes by various methods is a must in our daily life. Infection prevention by rinsing with water or washing off with soap and water is a common process to reduce the levels of microbial organisms on our skin. Numerous antimicrobial agents or materials, having varying water solubility and bioavailability to kill microbes, are also used in a wide range of concentrations and applications. Examples of such agents or materials include biocides, preservatives, antimicrobials and antibiotics. The mode of action for such agents can vary.

One method for controlling the growth and proliferation of microorganisms is to provide a controlled amount of an antimicrobial agent and have it constantly available to kill in the vicinity of the agent. The antimicrobial agent can be embedded or encapsulated in certain media with a specific release mechanism to ensure microbial kill for the protection of an underlying substrate or for the gradual release into an environment which needs to be protected from microbial attack over an extended period of time. From a biological test-method point of view, the antimicrobials form a kill zone or area around the media in which they are embedded or encapsulated that varies according to concentration and strength of efficacy of the antimicrobial. A certain amount leaches out constantly to provide a zone in which no organism can survive. The eluted amount must be above the Minimum Inhibiting Concentration (MIC). Usually a killing potential of around 95% is used to establish the MIC value of an antimicrobial. MIC values are commonly measured, to compare efficacy strength between different antimicrobials. The resulting area of no microbial growth is known as the "Zone of Inhibition."

Other terms used to describe antimicrobial function include bacteriostatic, fungistatic and biostatic. The definitions were in many cases overlapping with the terms bactericidal, fungicidal and biocidal. In general, however, the -cidal terms stand for eradicating or eliminating completely, whereas the -static terms stand for keeping the amount just in balance. Thus, -static refers to agents which kill organisms in an amount substantially equal to newly evolving organisms. From an MIC-value point of view, as discussed above, the value would be about 50% killing strength. However, the mode of action of an active chemical compound as bacteriostatic and bacteriocidal ingredient is still considered to be the same. U.S. Patent No. 2,510,428 discloses bacteriostatic and bacteriocidal concentrations ranging from 0.1ppm to 5% for 2, 3 diphenylindol, which relies on a concentration gradient for antimicrobial efficacy. GB 871228 discloses a biostatic plastic formed by extrusion of styrene/acylonitril containing chlorophenols. GB871228 states that antimicrobial efficacy is maintained after repeated washing and after years of use. The chlorophenols migrate to the surface of the plastic to provide biostatic activity. However, this forms a zone of inhibition around the surface of the plastic and the chlorophenols gradually deplete over time.

Wherever there is a free access of surfaces by microbial organisms, adherence of the organisms to such surfaces occurs and microbial contamination of these surfaces is a consequence. As a further consequence, it would be beneficial for numerous applications to prevent adherence of such organisms to a surface. Several methods for accomplishing this have been suggested. One way would be to constantly heat the surface to a temperature beyond the survival temperature of the organisms. This is not always practical or economical. Other ways of establishing an anti-microbial surface property that have been suggested include immobilizing antimicrobial, antiseptic or antibiotic agents on the surface of interest, for example, cellulosic, synthetic textile or medical device surfaces to reduce bacterial adhesion and subsequently prevent bacterial infection. The surfaces are prepared by entrapment or embedding of antimicrobial compounds in surface coatings. These surfaces involve a leaching mechanism and create a zone of inhibition. Chemically bonding (electrostatic, ionic or covalent) of active ingredients has also been suggested to achieve microbial adhesion prevention on surfaces of interest. However, in many cases the toxicological side effects are a concern, for example, in the case of covalent bonding of pentachlorophenol to a polymeric matrix. In most other cases, the antimicrobial efficacy is lost due to the synthesis of a different molecular entity.

Other attempts at immobilizing active ingredients to provide a non-leaching antimicrobial property that have been suggested include an ionic quat bonding mechanism, such as antimicrobial surface active polymers as discussed in U.S. Patent Nos. 4,229,838; 4,613,517; 4,678,660; 4,713,402; and 5,451,424. However, the ionic bonding drastically limits the longevity of efficacy of such surfaces. Over a relative short time in an aqueous environment, the ionically bonded antimicrobial moieties will be washed out. Additional examples of surface active polymers are discussed in U.S. Patent Nos. 5,783,502; 6,251,967; and 6,497,868, as well as in U.S. Published Application Nos. 2002/0051754, 2002/0177828, 2003/0175503 and 2003/117579. Although these references discuss reduced leaching of the active antimicrobial agent, they do disclose a covalent bonding mechanism or hydrophilic surface properties which provide long term efficacy for a non-leaching moiety. Further, there are other references that suggest the use of non-leaching active antimicrobial agents to provide an antimicrobial surface, but include a definition of "non-leaching" that would provide a zone of inhibition.

Antimicrobial surfaces employing long-chain antimicrobials with specific functional groups have also been proposed. As opposed to making antimicrobials available in solution, where organisms are attacked in free flowing aqueous or less mobile but moist environments with relative small biocidal molecular entities, it is suggested that the long chain antimicrobials provide killing surfaces by a different mode of action. The suggested mode of action involves the long chain molecular moieties penetrating the microbial cell. The pierced cell dies and the anchored long chain is ready for the next cell to be pierced. However, the prior art methods utilizing long chain antimicrobials have drawbacks which include significantly reduced efficacy over time due to insufficient bonding to the surface or a build-up of dead microbial bodies on the surface, and the formation of a zone of inhibition due to leaching or detachment of the penetrating moieties.

WO 02/10244 discloses biocidal polyurethane compositions wherein the polyurethane compound is at least partially end-capped with a group including at least one antimicrobial quaternary ammonium compound.

WO 99/33344 discloses biostatic compositions, as well as coatings thereof. The compositions contain a hydrophilic polymer such as, for example, a polyurethane polymer, possessing a functional group which covalently bonds to an amine, thiol, carboxyl or hydroxyl active group of an antimicrobial without reducing the antimicrobial property of the antimicrobial agent below its capability of acting as a biostatic agent and without releasing the antimicrobial agent into a solution.

EP 1 329 470 A1 discloses cationic amphiphilic associative polymers, a process for their preparation, their use as a thickener and a composition containing them.

US 5,084,096 discloses biocidal active surface coating compositions. The antimicrobial activity is not attributed to a slow release of the active quaternary ammonium compounds described therein, but rather to the active non-leachable surface.

It is an object of this invention to provide compositions which form durable coatings with long lasting antimicrobial efficacy without formation of a zone of inhibition and without the drawbacks discussed above.

Another object of this invention is to provide surface active antimicrobial coating compositions that include long chain molecules that chemically bond with a polymeric matrix upon drying or curing of the matrix to provide a non-leaching surface having long lasting antimicrobial efficacy.

It is another object of the invention to provide coatings in accordance with the preceding objects which are optionally hydrophilic and lubricious organic coatings which have good adherence to substrates, and, for applications involving contact with blood, to provide such coatings which do not trigger blood coagulation on the coated surfaces.

### SUMMARY OF INVENTION

The present invention provides a curable antimicrobial coating composition comprising:
(a) a polymeric matrix which comprises at least one polyurethane prepolymer, wherein said polyurethane prepolymer comprises a polyurethane backbone comprising at least one functional group capable of forming a chemical bond with the below-defined functional group of the long chain cationic surfactant, either directly or through a crosslinker, upon drying or curing of said coating composition;
(b) a carrier solvent;
(c) at least one long chain cationic surfactant compound comprising a functional group capable of forming a chemical bond with said polyurethane prepolymer upon evaporating said carrier solvent and drying or curing of said composition, said functional group being selected from the group consisting of an amine, thiol, carboxyl, aldehyde, hydroxyl and combinations thereof; and
(d) at least one hydrophilic organic monomer, oligomer, prepolymer, polymer or copolymer derived from vinyl alcohol, N-vinylpyrrolidone, N-vinyl lactam, acrylamide, amide, styrenesulfonic acid, combination of vinylbutyral and N-vinylpyrrolidone, hydroxyethyl methacrylate, acrylic acid, vinylmethyl ether, vinylpyridylium halide, methyl cellulose, ethyl cellulose, carboxymethyl cellulose, hydroxyethyl cellulose, hydroxypropyl cellulose, hydroxymethyl ethyl cellulose, hydroxypropylmethyl cellulose, cellulose acetate, cellulose nitrate, starch, gelatin, albumin, casein, gum, alginate, hydroxyethyl (meth)acrylate, hydroxypropyl (meth)acrylate, ethylene glycol (meth)acrylates (e.g. triethylene glycol (meth)acrylate) and meth)acrylamide), N-alkyl (meth) acrylamides (e.g. N-methyl (meth)acrylamide and N-hexyl (meth)acrylamide), N,N-dialkyl (meth)acrylamides (e.g. N,N-dimethyl (meth)acrylamide and poly-N,N-dipropyl (meth)acrylamide), N-hydroxyalkyl (meth)acrylamide polymers, such as poly-N-methylol (meth)acrylamide and poly-N-hydroxy ethyl (meth)acrylamide, and N,N-dihydroxyalkyl (meth)acrylamide polymers, such as poly-N,N-dihydroxyethyl (meth)acrylamide, ether polyols, polyethylene oxide, polypropylene oxide, and poly(vinyl ether), alkylvinyl sulfones, alkylvinylsulfone-acrylates or a combination thereof;
   wherein said at least one long chain cationic surfactant compound is non-leaching upon drying or curing of said coating composition and projects at least 15Å away from the surface of the cured coating composition to thereby protrude through and beyond organic debris deposited over time on the surface of said cured composition, wherein said organic debris are dead microbial cells, proteinaceous buildup or a combination thereof.

The aforesaid curable antimicrobial coating composition provides surfaces upon drying and evaporation of its carrier solvents with microbial, e.g., bacterial, adhesion prevention. A method of preparing and applying the composition of the present invention is also described herein. The mode of action is believed to be a microbial cell wall piercing mechanism without forming a zone of inhibition due to leaching. The reactive functional group(s) of the polyurethane prepolymer(s) underlying the polymeric matrix of the curable antimicrobial coating composition is reacted with counterparts of reactive group(s) of the antimicrobial long chain cationic surfactant compound(s) to form a new chemically, e.g. covalently, bonded, non-leaching polymeric matrix and converting the original antimicrobial potential based on leaching into an antimicrobial potential without leaching.

The piercing moieties of prepared surfaces are immobilized and do not leach out. The piercing moieties are preferably covalently bonded so that they are not subject of easy hydrolysis, which would allow the piercing moieties to be released and washed away. In terms of MIC, there is preferably no zone of inhibition formed and the MIC value is far below the 50% value, and is preferably close to or equal to zero. In practice, surfaces coated with the composition of the present invention, cured and exposed to micro-organisms, preferably do not exhibit a zone of inhibition, but still prevent growth or colonization of micro-organisms on treated surfaces.

The resulting non-leaching antimicrobial coated surfaces can be made optionally highly lubricous. Covalent links of the polymer to the antimicrobial can be established by the functions of esters, ethers, thioesters, thioethers, carbamates, urethanes, ureas, amides or linking mechanisms commonly used in polymerization, such as radical polymerization, or converting unsaturated carbon-carbon bonds into higher molecular branched single carbon-carbon bonds. The polymeric surface coating on a substrate with microbial adhesion prevention property provided by the cured coating composition of the present invention preferably withstands extensive exposure to a leaching solution without losing its antimicrobial property. The coated substrates preferably do not form a zone of inhibition as determined by bioassay. Suitable carrier solvents can include water, methyl ethyl ketone, N-methylpyrrolidone, tetrahydrofuran, ethyl lactate, dichloromethane, chloroform, ethyl acetate, propylene glycol methyl ether, propylene glycol methyl ether acetate, diacetone alcohol, ether, ester, aromatic hydrocarbons, chlorinated hydrocarbons, linear hydrocarbons and mixtures thereof. The composition is preferably useful for treating surfaces of medical devices, surgical dressings, hydrogels, textiles, paper, cloths, metals, glass, and plastics.

As noted herein above, the invention is directed to a curable antimicrobial coating composition comprising (a) the above-specified polymeric matrix, (b) a carrier solvent, (c) at least one long chain cationic surfactant compound comprising a functional group capable of forming a chemical bond with the polyurethane prepolymer(s) underlying the polymeric matrix upon evaporating the carrier solvent and drying or curing of the composition. The functional group of the long chain cationic surfactant is selected from the group consisting of an amine, thiol, carboxyl, aldehyde, hydroxyl and combinations thereof. The at least one long chain cationic surfactant compound is non-leaching upon drying or curing of the composition and is capable of penetrating cell walls of microbial organisms and preventing microbial colonization on the surface of the cured composition. The at least one long chain cationic surfactant compound also has sufficient length to protrude through organic debris deposited over time on the surface of the cured composition: to this end, the at least one long chain cationic surfactant projects at least 15Å away from the surface of the cured coating composition.

The polymeric matrix preferably includes at least one polyurethane prepolymer comprising at least one functional group capable of forming a chemical bond, preferably a covalent bond, with the functional group of the long chain cationic surfactant compound, either directly or through a cross-linker, upon drying or curing of the coating composition.

Preferably, the coating composition includes a combination of at least two long chain surfactants. The combination of at least two long chain surfactants can include surfactants having different chain lengths. Preferably, the cationic long chain surfactant is a quaternary ammonium compound.

The quaternary ammonium compound is preferably selected from the group consisting of an alkyl hydroxyethyl dimethyl ammonium chloride; polyquaternium 11; a quaternized copolymer of vinylpyrrolidone and dimethylaminoethylmethacrylate; polyquaternium 16; polyquaternium 44; a combination of a vinylpyrrolidone and quaternized vinylimidazol; polyquaternium-55; a quaternized copolymer of vinylpyrrolidone and dimethylaminoethyl; N,N-dimethyl-N-dodecyl-N-(2-hydroxy-3-sulfopropyl) ammonium betaine; N-alkyl acid amidopropyl-N,N-dimethyl-N-(3-sulfopropyl)-ammonium betaine; 3-chloro-2-hydroxypropyl-alkyl-dimethylammonium chloride with a long chain alkyl group; and combinations thereof.

Preferably, the long chain cationic surfactant projects at least 30 Å away and, most preferably, at least 60 Å away from the surface of the cured coating composition. Depending on the desired application and the thickness of the organic buildup, the surfactant can be chosen to adjust the distance that it projects away from the surface of the cured coating composition and beyond the organic debris. As noted herein above, the organic debris are dead microbial cells, proteinaceous buildup and a combination thereof.

Preferably, the curable coating composition includes a hydrophilic watersoluble organic monomer, oligomer, prepolymer, polymer or copolymer of a type and in an amount sufficient to provide the cured composition with a reduction in friction of at least about 70% compared to the uncoated surface when each are wetted with water or an aqueous solution. Preferably, the reduction in friction is at least about 80%, more preferably at least about 90% and, most preferably, at least about 95%.

The curable antimicrobial coating composition preferably comprises from 0.01% to 20% of said at least one polyurethane prepolymer based on the weight of the composition; at least one carrier solvent capable of at least partially dissolving said polyurethane prepolymer, present in an amount from 99.89% to 75% based on the weight of the composition; a hydrophilic component as defined herein above present in an amount from 0.01 to 40 % based on the weight of the composition; and at least one quaternary ammonium compound present in an amount from 0.01% to 5% based on the weight of the composition and selected from the group consisting of an alkyl hydroxyethyl dimethyl ammonium chloride; polyquaternium 11; a quaternized copolymer of vinylpyrrolidone and dimethylaminoethylmethacrylate; polyquaternium 16; polyquaternium 44; a combination of a vinylpyrrolidone and quaternized vinylimidazol; polyquaternium-55; a quaternized copolymer of vinylpyrrolidone and dimethylaminoethyl; N,N-dimethyl-N-dodecyl-N-(2-hydroxy-3-sulfopropyl) ammonium betaine; N-alkyl acid amidopropyl-N,N-dimethyl-N-(3-sulfopropyl)-ammonium betaine; 3-chloro-2-hydroxypropyl-alkyl-dimethylammonium chloride with a long chain alkyl group; and combinations thereof.

In one embodiment, the polyurethane prepolymer contains at least one functional group selected from the group consisting of a reactive isocyanate, blocked isocyanate, thioisocyanate, carboxyl, amino, vinyl and combinations thereof. Preferably, the at least one functional group is selected from the group consisting of a reactive isocyanate, blocked isocyanate and thioisocyanate.

The aforesaid coating composition can also include a modifying polymer selected from the group consisting of polyester, polyalkyd, maleic anhydride polymer, maleic anhydride copolymer, polyol, polyamine, polyamid, polyacrylate, polyvinyl alcohol, polyvinyl acetate, polyglucosamid, polyglucosamine, polyvinylpyrrolidone, their copolymers and combinations thereof.

Preferably, the hydrophilic component in the aforesaid coating composition is present in an amount from 0.2% to 15% and, more preferably, 1% to 12%, based on the weight of the composition in replacement of the carrier solvent. The hydrophilic polymer, copolymer or prepolymer is most preferably polyvinylpyrrolidone (PVP). Preferably, the PVP is present in an amount at least approximately equal to the amount of the quaternary ammonium compound.

In the case where a crosslinker is used, the crosslinker is preferably selected from the group consisting of an aziridine, carbdiimid, melamine, a substituted melamine, a melamine derivative, multifunctional alcohol, multifunctional aldehyde, multifunctional amine, multifunctional isocyanate and combinations thereof. The crosslinker is preferably present in the aforesaid coating composition in an amount from 0.001% to 5%, and more preferably 0.1% to 2.5%, based on the weight of the composition in replacement of said carrier solvent.

The aforesaid coating composition can also include a reaction enhancing catalyst. Preferred catalysts include catalysts selected from the group consisting of tin organic compounds, cobalt organic compounds, trimethylamine, triethylamine and combinations thereof. Examples of preferred catalysts include dibutyltin dilaurate and cobalt octoate.

The carrier solvent can be selected from the group consisting of water, methyl ethyl ketone, N-methylpyrrolidone, tetrahydrofuran, dichloromethane, chloroform, ethyl acetate, propylene glycol methyl ether, propylene glycol methyl ether actetate, diacetone alcohol, ether, ester, aromatic hydrocarbon, chlorinated hydrocarbon, linear hydrocarbon and combinations thereof.

Preferably, the aforesaid coating composition contains a combination of at least two of the above-listed quaternary ammonium compounds. In one preferred embodiment, the aforesaid coating composition contains a combination of a 3-chloro-2-hydroxypropyl-stearyl dimethyl ammonium chloride and an alkyl hydroxyethyl dimethyl -R- ammonium chloride. In one embodiment, the aforesaid coating composition contains a combination of at least three of the above-listed quaternary ammonium compounds. In such an embodiment, the combination preferably includes an alkyl hydroxyethyl dimethyl ammonium chloride, a 3-chloro-2-hydroxypropyl-cocoalkyl-dimethyl ammonium chloride and a 3-chloro-2-hydroxypropyl-stearyl-dimethyl ammonium chloride, e.g., a combination of alkyl hydroxyethyl dimethyl R ammonium chloride (R=C12) [Praepagen HY (Clarient)], Quab 360 (Degussa) and Quab 426 (Degussa).

The aforesaid coating composition can also include an additional component intended to leach out of the cured coating composition selected from the group consisting of an antimicrobial compound, biocide, antibiotic, drug, vitamin, fungicide, fungistat, virucide, germicide, spermacide, therapeutic agent, plant extract and combinations thereof.

Described herein is a medical device for introduction into a human or animal body, comprising an antimicrobial coating on at least one surface of the device, the antimicrobial coating being the cured coating composition of the present invention.

Additional objects, advantages and novel features of the invention will be set forth in part in the description and examples which follow, and in part will become apparent to those skilled in the art upon examination of the following, or may be learned by practice of the invention. The objects and advantages of the invention may be realized and attained by means of the instrumentalities and combinations particularly pointed out in the appended claims.

### DETAILED DESCRIPTION OF THE INVENTION

The present invention relates to a non-leaching, antimicrobial coating composition providing surfaces upon drying and evaporation of the carrier solvents of the composition with a bacteria adhesion prevention surface coating.

As used in the specification and claims hereof, the following terms have the particular meanings and definitions set forth below:
The term "chemical bond" as used herein is meant to be interpreted broadly to encompass not only covalent bonding and ionic bonding, but also interactions, such as, for example, van der Waals forces and hydrogen bonding to the degree that they can not be overcome by hydrolytic interaction with water so as to cause the originally linked antimicrobial to become leachable and form a cleaved antimicrobial entity that creates a zone of inhibition.

The term "antimicrobial" as used herein is meant to include a material that engages in a biological activity or which is effective against microorganisms. Antimicrobial moieties suitable for use in the present invention are long chain cationic surfactants and possibly, in addition thereto, anionic and non-ionic surfactants that provide, after curing the coating composition, an antimicrobial, non-leaching durable film, which functions without formation of a zone of inhibition due to leaching.

The coating composition according to the present invention includes a polymeric matrix containing functional groups that can bond covalently with amine, thiol, carboxyl, aldehyde or hydroxyl active groups of selected long chain cationic surfactant compounds. The length of the selected long chain cationic surfactant compounds are long enough to project at least 15Å away from the surface of the cured coating composition to thereby protrude through organic debris deposited over time on the resulting coating during use. These long chain compounds become non-leaching upon curing of the coating composition and are capable of penetrating cell walls of microbial organisms and disrupting cell functional activities to prevent microbial colonization on the coated surface.

The long chain antimicrobials can include either an unsubstituted amine moiety, a thiol, a carboxyl, a hydroxyl moiety, an aldehyde or any possible combination of any one or more of these moieties alone or in combination. In addition, the term "antimicrobial molecule" as used herein may mean any one or more of an antimicrobial molecule alone or a combination of different antimicrobials. Furthermore the unsubstituted amine function of the antimicrobial may serve as starting function to modulate into more reactive isocyanate function by known reaction with phosgene or phosgene derivatives. In general the individual functional group can either be present at the polymeric backbone, the crosslinker or the antimicrobial to complement the functional group without limitation of the position in the polymeric matrix or in the antimicrobial moiety.

The term non-leachable as used herein means that the coating is no longer releasing quantities of an original antimicrobial moiety in concentrations that are biologically active, i.e., they are not biocidal anymore in terms of a zone of inhibition. The leach-out concentrations are below the actual efficacy levels in an aqueous solution and therefore do not control microbial growth. Test samples coated with curable antimicrobial coating compositions of the present invention were subjected to extensive leaching in the presence of saline solution or demineralized water for at least 28 days prior to biological testing. The coatings did not lose their efficacy after the 28-day leaching cycle, confirming that the antimicrobial moiety was bonded to the surface. The non-leaching antimicrobial status, after the 28-day leaching cycle, was confirmed by microbial testing when a.) no zone of inhibition is detected and b.) no adhesion or growth of microbes was evident after 24 hrs of microbial exposure and 5 days of incubation time of the leached surfaces which were coated with the curable antimicrobial coating compositions according to the present invention.

The antimicrobial curable coating compositions according to the present invention, upon drying and curing, provide a non-leaching antimicrobial surface with long term efficacy against a target microorganism for, preferably, at least about 3 months. Preferably, the efficacy is maintained for at least about 6 months, more preferably at least about 9 months and, most preferably, at least about 1 year. The target microorganisms can include Escherichia coli and/or Staphylococcus aureus.

As described herein above, in one embodiment, the reactive functional group(s) of the polyurethane prepolymer(s) underlying the polymeric matrix of the curable antimicrobial coating composition is reacted with counterparts of reactive group(s) of the antimicrobial long chain cationic surfactant compound(s) to form a new covalently bonded moiety in a non-leaching polymeric matrix by converting the original antimicrobial into an antimicrobial surface active polymeric coating which does not have a mode of action based on a leaching. In another embodiment, the covalent links can be established by crosslinkers. Thus, the covalent links of the polymer to the antimicrobial can be establish by the functions of esters, ethers, thioesters, thioethers, carbamates, urethanes, ureas, amides or linking mechanisms commonly used in polymerization such as radical polymerization or converting unsaturated carbon-carbon bonds into higher molecular branched single carbon-carbon bonds or by the use of crosslinkers. The resulting non-leaching anti-microbial coated surfaces can be made optionally highly lubricous.

The so-modified antimicrobial polymers may coat sheeting materials made of polycarbonate, PVC, polyurethane, glass, and ceramic. The resulting surface is not only antimicrobial without forming a zone of inhibition (no leaching), but also has anti-fog and anti-frost properties. Uses for such coatings include greenhouses, clean room walls, walls of food handling rooms, and freezer doors.

To form the non-leaching antimicrobial surface coating polymers which immobilize the antimicrobial long chain cationic surfactant compound(s), the antimicrobial can be bonded to the polyurethane through a crosslinker. Crosslinkers suitable for immobilizing the antimicrobial, and capable of forming an antimicrobial polymeric surface, include multifunctional molecules with at least two functionalities of isocyanates, carboxyl groups, acrylic acid derivatives, aldehyde groups, alcohol groups, aziridines or carbodiimid. The semi-crosslinked composition material may be employed as an antimicrobial polymeric material or as an antimicrobial coating. It becomes fully crosslinked upon drying and curing. In addition, such crosslinked materials may be further modified to contain optionally additional antimicrobials, antibiotics or drugs not subject to complete immobilization, covalent bonding or crosslinking with the aforementioned crosslinker for the purpose of an intentional and controlled elusion for supportive antimicrobial or therapeutic performance.

The preferred method of linking antimicrobial long chain cationic surfactant compounds, suitable for a non-leaching antimicrobial mode of action, is the formation of a covalent bond by reacting an available free isocyanate group from a polyurethane prepolymer with an amine or hydroxyl group of specific antimicrobial quaternary ammonium compounds which have long chain molecular moieties. Ionic bonding or other chemical interaction are only useful for the curable coating compositions of the present invention if microbial free surfaces are detected according to the afore mentioned definition of "non-leachable."

It has been discovered that not all quaternary ammonium compounds have the desired property of non-leaching and simultaneously maintaining the non-adhering antimicrobial efficacy. Surprisingly, it was found that a quaternary ammonium compound having the formula below meets these requirements: 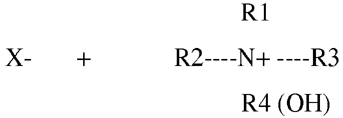 wherein at least one of the groups R1, R2 or R3 has a length sufficient to penetrate cell walls of microbial organisms, so as to kill the cells and prevent microbial colonization over the surface of the cured compositions; and R4 has a length sufficient so that at least one of the other R groups protrudes through organic debris deposited over time on the surface of the cured composition and the OH-functional group on R4 will covalently bond to the polymeric matrix of the coating composition upon drying or curing of the composition. Preferably, R4 has a length sufficient so that N is at or protrudes through any organic debris deposited over time on the surface of the cured composition. Additionally, the R4 group may contain reaction enhancing groups in the alpha position to the reactive group in R4. These suitable quaternary ammonium compounds with reaction groups dissolved in water are used for covalent bonding to residual isocyanate containing polyurethanes contained in the polymeric matrix of the composition.

Suitable quaternary ammonium compounds have three important designs: (a) they contain a functional group such as primary amine, hydroxyl or thiol groups to be able to react with the residual isocyanate group of the PU prepolymer to form a urea, carbamate and thiocarbamate respectively; (b) the carbon chain with the isocyanate reacting functional group is long enough to allow the quaternary compound to protrude through any proteinacious build-up; and (c) the compound contains at least one additional carbon chain capable of piercing the cell wall of the microbial organisms. In one embodiment, the additional carbon chain is 13 carbon atoms or higher.

The at least one quaternary ammonium compound is preferably selected from the group consisting of an alkyl hydroxyethyl dimethyl ammonium chloride (Praepagen HY from Clarient), polyquaternium 11, a quaternized copolymer of vinylpyrrolidone and dimethylaminoethylmethacrylate, polyquaternium 16, polyquaternium 44 (vinylpyrrolidone and quaternized vinyl imidazol), polyquaternium 55 (quaternized copolymer of vinylpyrrolidone and dimethylaminoethyl), N,N-dimethyl-N-dodecyl-N-(2-hydroxy-3-sulfopropyl) ammonium betaine (Ralufon DL-OH), N-alkyl acid amidopropyl-N,N-dimethyl-N-(3-sulfopropyl)-ammonium betaine (Ralufon CAS-OH) and 3-chloro-2-hydroxypropyl-alkyl-dimethylammonium chloride with a long chain alkyl group. Preferred long chain alkyl groups include dodecyl (e.g., Quab 342 from Degussa), cocoalkyl (e.g., Quab 360 from Degussa) and/or stearyl (e.g., Quab 426 from Degussa).

Preferably, the curable coating composition contains a combination of at least two of the above-listed quaternary ammonium compounds. Preferred combinations include the following: (1) Ralufon DL-OH and Quab 360; (2) Praepagen HY and Quab 426; (3) Quab 342 and Ralufon CAS-OH; and (4) Praepagen HY and Quab 360. More preferably, the coating composition contains a combination of 3-chloro-2-hydroxypropyl-stearyl dimethyl ammonium chloride (Quab 426 from Degussa) and alkyl hydroxyethyl dimethyl -R- ammonium chloride (Preapagen HY from Clarient). Preferably, the combinations of quaternary compounds are included in the ratio of about 3:1 to about 1:3 relative to each other.

As previously indicated herein above, the curable coating composition also includes a hydrophilic organic monomer, oligomer, prepolymer, polymer or copolymer derived from vinyl alcohol, N-vinylpyrrolidone, N-vinyl lactam, acrylamide, amide, styrenesulfonic acid, combination of vinylbutyral and N-vinylpyrrolidone, hydroxyethyl methacrylate, acrylic acid, vinylmethyl ether, vinylpyridylium halide, methyl cellulose, ethyl cellulose, carboxymethyl cellulose, hydroxyethyl cellulose, hydroxypropyl cellulose, hydroxymethyl ethyl cellulose, hydroxypropylmethyl cellulose, cellulose acetate, cellulose nitrate, starch, gelatin, albumin, casein, gum, alginate, hydroxyethyl (meth)acrylate, hydroxypropyl (meth)acrylate, ethylene glycol (meth)acrylates (e.g. triethylene glycol (meth)acrylate) and meth)acrylamide), N-alkyl (meth) acrylamides (e.g. N-methyl (meth)acrylamide and N-hexyl (meth)acrylamide), N,N-dialkyl (meth)acrylamides (e.g. N,N-dimethyl (meth)acrylamide and poly-N,N-dipropyl (meth)acrylamide), N-hydroxyalkyl (meth)acrylamide polymers, such as poly-N-methylol (meth)acrylamide and poly-N-hydroxy ethyl (meth)acrylamide, and N,N-dihydroxyalkyl (meth)acrylamide polymers, such as poly-N,N-dihydroxyethyl (meth)acrylamide, ether polyols, polyethylene oxide, polypropylene oxide, and poly(vinyl ether), alkylvinyl sulfones, alkylvinylsulfone-acrylates or a combination thereof.

Preferably, the hydrophilic polymer, copolymer or prepolymer is present in an amount from 0.1% to 40%, and more preferably from 0.2% to 15%, based on the weight of the composition in replacement of the carrier solvent. The hydrophilic polymer, copolymer or prepolymer is most preferably polyvinylpyrrolidone (PVP).

In regard to the combination of a polyurethane, a quaternary ammonium compound and a carrier solvent, as discussed above, it is believed that the hydrophilic polymers unexpectedly enhance the performance of the antimicrobial coating. It was discovered that some quaternary ammonium containing coatings required a certain amount of PVP to assure proper activation when the cured coating is transferred into a hydrolyzed and activated coating. The amount of PVP required can be at least about an equivalent amount to the quaternary compound before a noticeable lubricity is achieved.

The preferred PVP concentration is 0.1 to 5% of the coating composition, where no specific lubricity is intended. The preferred PVP concentration is 2 to 12% of the coating composition, where high lubricity is intended.

While not being bound by theory, it is believed that the dipole-dipole interaction between the hydrophilic polymer and water is needed to penetrate along the PVP complex to orient the quaternary ammonium complex into an upright position. This is believed to enhance the antimicrobial function of the cured composition by orienting the antimicrobial compound to project away from the surface of the cured coating.

In one embodiment, the curable coating composition can also include at least one auxiliary agent for performance enhancement of the coating composition and/or the resulting coating on the coated surface.

Preferably, the auxiliary agent is selected from a surfactant or wetting agent, emulsifier, dye, pigment, colorant, UV absorber, radical scavenger, anti-oxidant, radical initiator, anti-corrosion agent, optical brightener, reactive or tracer fluorescer, bleaches, bleach activators, bleach catalysts, non-activated enzymes, enzyme stabilizing systems, chelants, coating aid, metal catalyst, metal oxide catalyst, organometallic catalyst, film forming promoter, hardener, linking accelerator, flow agent, leveling agent, defoaming agent, lubricant, matte particle, rheological modifier, thickener, conductive or non-conductive metal oxide particle, magnetic particle, antistatic agent, pH control agents, perfumes, preservative, biocide, pesticide, anti-fouling agent, algicide, bactericide, germicides, disinfectant, fungicide, bio-effecting agent, vitamin, drug, therapeutic agent or a combination thereof.

In one embodiment, the concentration of the auxiliary agent for performance enhancing is from 0.001% to 10%, preferable from 0.01% to 5%, based upon the weight of the coating composition.

In one embodiment, the curable coating composition contains an organic solvent in an amount of from 0% to 50% and water in an amount of from 0.5% to 95%, preferably 1% to 50% by weight.

The curable coating composition can be coated onto the surface of an object selected from the group consisting of a metal, metal alloy, plastic, glass, human skin, animal skin or fibrous material. The object can also be a medical device for introduction into a human or animal body, wherein the cured coating composition is included on at least one surface of the device.

The medical device can be at least partially made of a metal or metal alloy consisting of stainless steel, nickel, nickel-cobalt, titanium, NiTi, tantalum, nitinol, rare earth metal, silver, gold, platinum, tungsten, combinations thereof or alloys or plated articles thereof.

The medical device can be at least partially made of polyurethane, polycarbonate, polyethers, polyesters, polyvinyl chloride, polystyrene, polyethylene, polypropylene, polyvinyl acetate, silicone rubbers, rubber latex, polyester-polyether copolymers, ethylene methacrylates, silicone, natural and synthetic rubbers, nylon, PEBAX, polyamide or combinations thereof.

The medical device can be at least partially made of glass such as optical glasses, optical lenses, polarizing glasses, mirrors, optical mirrors, prisms, quartz glass and the like.

The medical device may be coated by a coating composition of the present invention by dipping, brushing, flooding, spraying, bar coating, roll coating, electrolytic depositing, electrostatic spraying, electroplating, vacuum treatment, pressure treatment or combinations thereof.

The medical device can be in the form of a tube, capillary, wire, sheet, coil, rod, lattice or network of wires.

The medical device can be a surgical rod, an orthopedic implant, a guidewire, a guidewire tubing, a coiled guiding tube, a coiled catheter, an expendable or non-expendable stent, an electrodal coil, a needle, a blade, a pace maker or similar metallic medical device.

The medical device can also be a tablet, a capsule, tubing, a capillary, a sheet, a fiber, a wound dressing, a tissue separator, a suture thread, a balloon, a foil, a catheter, a dialysis catheter, a urinary catheter, a guiding tube, a wound drain, a stent or a similar medical device.

The auxiliary agent may optionally be chemically bonded and/or physically incorporated into the curable coating composition or incorporated into the finished coating on the surface of the object.

The auxiliary agent may optionally be a preservative selected from the group consisting of parabens, formaldehyde releasers, haloalkyls, haloalkynyls, alkyl acids, aryl acids, isothiazolinons, quats, zinc oxide, zinc organics, iodine, povidone-iodine, chlorhexidine, bronopol, triclosan, clotrimazol, miconazole, propiconazole, tebuconazole, tolnaphtate, clioquinol, colloidal silver, silver complexes and silver salts or combinations thereof.

The auxiliary agent may optionally be an antimicrobial agent selected from the group consisting of antibiotics, antiseptics, disinfectants including tetracyclines, rifamycins, rapamycin, macrolides, penicilins, cephalosporins, beta-lactam antibiotics, aminoglycosides, chloramphenicol, sufonamides, glycopeptides, quinolones, ciprofloxacin, fusidic acid, trimethoprim, metronidazole, clindamycin, mupirocin, polyenes, azotes, fluconazole, and beta-lactam inhibitors.

The auxiliary agent may optionally be a therapeutical agent selected from the group consisting of analgesics, anti-inflammatory agents, topical antipuritics, anti-itch, non-steroids, acetaminophen, ethylsalicylic ester, camphor, bufexamac, ibuprofen, indomethacin, steroids such as hydrocortisone, desonide, triamcinolone acetonide, betamethasone valerate, betamethasone dipropionate, betamethasone benzoate, clobetasol propionate, halcinonide, desoximethasone, amcinonide, fluocinonide, fluandrenolide, alclometasone dipropionate, fluocinolone acetonide, diflorasone diacetate, mometasone furoate, fluorometholone, clocortolone pivalate, triamcinolone acetonide, halcinonide, dermatological agents, anthralin coal tar extract, keratolytic agent salicylic acid, urea, a local anaesthetic agent such as lidocaine, benzocaine, an anti-acne agent such as benzoyl peroxide, vitamin A derivatives, a wart removing agent such as salicylic acid, and lactic acid; and other like agents and cyclodextrin complexes thereof.

The auxiliary agent may optionally be a drug selected from the group consisting of an anti-thrombogenic drug, or anti-thrombogenic agent, or stent restinosis preventing drug, including taxol, paclitaxel, paclitaxel derivatives, dexamethasone and derivatives, heparin and its derivatives, aspirin and hirudin, a nitric oxid drug derivative, a nitric oxide releasing drug, tacrolimus, everolimus, cyclosporins, sirolimus, angiopeptin and enoxaprin or combinations thereof.

The auxiliary agent may optionally be a radiopaque compound selected from the group consisting of diatrizoate, iothalamate, metrizoate, iodipamide, triiodobenzoic acid, iothalamic acid, iopanoic acid, triiodophenyl acid, iodothalamic acid, iodine, iodides, bromine, perfluorooctyl bromide, barium sulfate samarium, erbium, bismuth salts (including oxy salts and oxides), titanium oxide, zirconium oxide, gold, platinum, silver, tantalum, niobium, tungsten, gold, titanium, iridium, platinum or rhenium and combinations thereof.

The metal or metal alloy object can be made of a metal or metal alloys selected from the group consisting of aluminum, magnesium, beryllium, iron, zinc, stainless steel, nickel, nickel-cobalt, chromium, titanium, tantalum, rare earth metal, silver, gold, platinum, tungsten, vanadium, copper, brass, and bronze or combinations thereof or plated articles thereof.

The plastic objects can be made of polymers selected from the group consisting of transparent or non-transparent polyurethane, polycarbonate, polyethers, polyesters, polyvinyl chloride, polystyrene, polyethylene, polypropylene, polyvinyl acetate, silicone rubbers, rubber latex, polyester-polyether copolymers, ethylene methacrylates, silicone, natural and synthetic rubbers, nylon, polyamide or combinations thereof.

The glass objects can be at least partially made of glass, such as optical glasses, optical lenses, polarizing glasses, mirrors, optical mirrors, prisms, quartz glass, and ceramics.

The plastic objects can include face shields, helmet shields, swim goggles, surgeon face shields, food packaging plastic foil, greenhouse walls, greenhouse roofs, mirrors, wind shields, underwater moving objects, airplane window shields, passenger air-balloons, gloves, aprons, and sponges.

The glass objects can include window glasses, greenhouse glasses, glass sheets, face shields, optical glasses, optical lenses, polarizing glasses, mirrors, optical mirrors, prisms, quartz glass, parabolic antennas, automobile head beam light glasses, automobile windshields, airplane control light glasses, and runway lights.

The fibrous material can contain metal, glass, plastic or cellulose, and can include polymeric materials in the form of filters to prevent air born microbial contamination (e.g., woven and non-woven materials, cast membranes over such materials, spun bonded materials and electro-spun materials), textiles such a clothing, tents for the purpose of preventing microbial colonization in a self decontamination process.

The presently described curable antimicrobial coating compositions are suitable for applications such as: a) Treatment of surfaces of medical devices; b) Treatment of surfaces in medical, dental and veterinary operation rooms; c) Treatment of general hygiene care requiring surfaces in households; d) Treatment of surfaces in nurseries and day care facilities; e) Treatment of surfaces of consumer goods; f) Treatment of surfaces in food processing industries, cosmetic manufacturing and the like; g) Treatment of food packaging materials; h) Treatment of surfaces of agricultural uses, e.g. in seed treatments, animal care etc.; and i) Treatment of industrial products, chemicals, pigments, inks, dyes, resins, adhesives, textiles, paper, leather, wood, plaster, and other treatment requiring surfaces.

The curable antimicrobial coating composition of the present invention can be used to prepare, inter alia, agricultural products, cleaning compositions, antimicrobial sponges, antimicrobial bleaching agents, antimicrobial fillers for paints, plastics, or concrete, and to treat concrete structures such as livestock shelters, where microbial infestation is a problem.

Surfaces and substrates treatable with the curable antimicrobial coating compositions of the present invention include, but are not limited to, textiles, carpet, carpet backing, upholstery, clothing, sponges, plastics, metals, medical devices of silione, polyurethane, PVC and the like for drainage tubing, dialysis and urinary catheters, biliary tubings and biliary stents, feeding tubes, medial hydrogels, topical and transdermal carrier applications, biodegradable hydrogels with topical and internal applications, surgical dressings, anti-mirobial anti-fog sheets, greenhouse sheeting, freezer doors, masonry, silica, sand, alumina, aluminum chlorohydrate, titanium dioxide, calcium carbonate, wood, glass beads, containers, tiles, floors, curtains, marine products, tents, backpacks, roofing, siding, fencing, trim, insulation, wall-board, trash receptacles, outdoor gear, water purification systems, and soil. Furthermore, articles treatable with the curable antimicrobial coating compositions of the present invention include, but are not limited to, air filters and materials used for the manufacture thereof, aquarium filters, buffer pads, fiberfill for upholstery, fiberglass duct-board, underwear and outerwear apparel, polyurethane and polyethylene foam, sand bags, tarpaulins, sails, ropes, shoes, socks, towels, disposal wipes, hosiery, feminine hygiene products and intimate apparel; cosmetics, lotions, creams, ointments, disinfectant sanitizers, wood preservatives, plastics, adhesives, paints, pulp, paper, cooling water, and laundry additives and non-food or food contacting surfaces in general. Other examples include general odor control in clothing, antimicrobial band aid design, protective barrier materials in animal care including mastitis control, clean room design and wall treatments in food handling rooms.

Cured coatings of the curable antimicrobial coating composition of the present invention can also be suitable in military applications, such as protection against biological warfare, self-decontamination of war planes, cargo and shipping boxes, envelopes, uniforms, and army ducts.

Moreover, after treating a surface or fabric with the curable antimicrobial coating compositions of the present invention, the surface or fabric may, optionally, be heated to further complete cross linking and bonding of the composition to the surface or substrate upon evaporation of carrier solvents.

Treating food crops (e.g., perishables such as vegetables, fruits, or grains) in a pre- or post-harvest process with the compositions of the present invention imparts antimicrobial protection to the outer surface of the food crop. It is believed that such protection occurs without diffusing, migrating or leaching the antimicrobial agent from the bonded antimicrobial coating of the food item, and provides prolonged, safe and non-toxic antimicrobial protection. The method involves treating fruits and vegetables in the rinse cycle, during or after the normal cleaning/water spraying or during or after blanching. Thorough cleaning of fruits and vegetables at the processing plant is preferred for initially removing microorganisms. As one of ordinary skill in the art would recognize, machines are used initially to remove soil, chemicals used in growing, spoilage bacteria, and other foreign materials. These machines also use high velocity water sprays to clean the products. After the cleaning, raw foods or other crop materials are prepared for further processing such as blanching (i.e., the food is immersed in water at 87.8 to 98.9 °C (190 to 210 °F) or exposed to steam).

Treating surgical gloves with the curable antimicrobial coating compositions of the present invention before or during a surgical procedure can prevent colonization and cross contamination. It is believed that the treated gloves provide prolonged antimicrobial activity with safe and non-toxic antimicrobial protection. Surgical gloves are treated, preferably, by submerging in a curable coating composition of the present invention. This method will permit doctors to use the gloves with lower risk of cross contamination.

Moreover, one of ordinary skill in the art would be able to implement numerous other end uses based upon the present disclosure. For instance, the following uses, applications and substrates, are also contemplated as being particularly preferred: treating orthopedic implants, skin or other tissues (bone, soft tissues) for use in a transplant to reduce microbial contamination. The composition is likewise useful in any toothpaste formulation known in the art to enhance the caries-fighting properties of such compositions through anti-microbial treatment of teeth.

The preferred embodiments underlying the present invention are set forth in the examples below. Other features of the present invention will become apparent from the following examples, which are for illustrative purposes only and are not intended as a limitation upon the present invention.

The curable antimicrobial coating composition of the present invention has a number of advantages over conventional biocide eluting coatings, as well as over the alleged bacteriostatic, non-eluting compositions of prior art. The advantageous properties of the anti-bacterial coating composition of the present invention after curing are:
the resulting coating film does not leach-out any anti-microbial agent; the anti-microbial agent is immobilized by the coating polymeric matrix; the resulting coating film has a long lasting efficacy against microbes; the resulting coating film, with its non-leaching mode of action, has no side effects or secondary toxicity, which is important for products requiring regulatory approval; and the resulting coating film can optionally be lubricous for a wide variety of applications in medical, veterinarian, food packaging, textile, polymeric fabric, household, personal care, consumer goods, anti-fog, construction, agricultural and other applications.

Additional testing of the molecular and cell-biological impact was also evaluated. The cured coating of a curable antimicrobial coating composition according to the present invention did not reveal a cytotoxicity potential according to standard test method ISO 100993, part 5. Exposure to protein solution did not reveal a compromise in long-term, non-leaching antimicrobial performance. These findings are particularly important when a curable antimicrobial coating composition of the present invention is applied in the medical area where tissue contact is involved, as well as when in contact with food-protein or body protein.

Blood contact tests surprisingly revealed an impact on the coagulation speed where blood is brought into contact with surfaces, treated with the curable antimicrobial coating composition according to the present invention. The blood tends to coagulate slower or not at all when in contact with a so-treated surface.

With a dynamic test procedure simulating the flow rate of a bile solution containing microbes, it was discovered that over at least one week there was no slime or biofilm build up on a surface coated with an antimicrobial coating composition underlying the present invention. Uncoated samples and samples with lubricious coating (without the antimicrobial compound) showed biofilm formation in this dynamic test, within one week.

### Experimental

### Leaching Procedure

Curable antimicrobial coating compositions according to the present invention were coated onto 2cm by 2cm polyurethane test samples on one side, air-dried for about 10 minutes and then oven-dried and cured at elevated temperature around 50 to 95 °C for about 30 min. The cured samples were subject to washing in phosphate buffer solution (PBS) for 1, 7, 14, 21 and 28 days, and for 2 and 3 months and longer at about 23 °C. The samples were placed in 100ml leaching solution of PBS. After brief shaking the 100ml leaching solution was replaced once every week. After each time interval the samples were rinsed 3 times in 5ml of demineralized water, dried for 10 min at room temperature and then subject to microbial testing.

### Coating Solution Preparation

Coating solutions containing PU, and optionally PVP, according to the prior art were prepared. To these solutions was added 10% of a polyurethane prepolymer containing about 6% free isocyanate groups measured by titration prior to the addition.

The percentage isocyanate concentration present in the polyurethane prepolymer was determined with 25ml of a 0.1 N dibutyl amine solution (slight excess of expected amount) and mixed for 15 minutes. The excess was titrated back with 0.1 n HCl against a bromophenol blue indicator until faint yellow was seen.

### Preparation and Use of Coating Solutions

The free isocyanate containing coating solutions were briefly mixed and then 5% to 15% of the 40 to 90% aqueous solutions of quaternary ammonium compounds (containing an active functional group in accordance with the present invention) were added and briefly mixed again. The mixture was left for observation in a first evaluation for reactivity. The mixtures were observed to gel in about 2 to 4 hours, indicating a slow reaction speed, which gives time for the actual coating process.

Further samples of coating solutions with reactive functional group-containing antimicrobials with long carbon-carbon chains according to the present invention were prepared in a similar way. The final coating solution was applied immediately after mixing of the additional isocyanate containing polyurethane prepolymer and the reactive functional group-containing antimicrobials for about 15 minutes. The coatings had good adhesion and did not deteriorate in the presence of water or PBS. Some of the samples had lubricous properties.

Surprisingly, it was found that, despite of the presence of water, there is sufficient interaction with the competition reaction of the residual isocyanate and the primary amine, hydroxyl and thiol function of the antimicrobial. It was also found that the final composition has a pot life of a few hours, depending on temperature, reactive functional group of antimicrobial and possible catalytic interaction. The reactive coating composition is applied to a variety of substrates, cured and subsequently washed with water to remove any excess of unreacted antimicrobial. It was repeated several times with fresh PBS on a weekly basis to assure complete removal.

### Microbial Testing

Bacterial suspension of E. coli and Ps. aeruginosa and St. aureus with 1 x 10⁶ cells/ml each in sterile buffer solution were prepared for microbial exposure. 25µl of the suspension were dropped onto the sample inside a Petri dish and immediately covered with agar plates. The dish was closed, sealed and incubated at 37 °C for 24 hours. After incubation the bacterial growth of colonies were counted after 5 days in the closed dish avoiding the agar to get dry. Colony counts were recorded numerically and by microphotographs to show extent of microbial growth for samples and controls for each organism after each week of the total leaching period. The bacteria tests are performed at 37°C and allowed 24 hours to grow on the polyurethane coated surface. A bacteria pellet supplied by MicroBioLogics (ATCC # 25922 for E. coli and ATCC # 29213 for S. aureus) was cultured in 5 ml of LB Broth solution and allowed to incubate for 4 hours before 40µl were pipetted onto the coated polyurethane surface. Results were viewed with a 20X microscope.

### Examples:

### Controls

Formulations according to patents US 4,467,073, US 4,642,267 and US 6,054,504 were used as controls containing no antimicrobial with and without additional polyurethane prepolymer containing additional isocyanate groups.

### Uncoated Sample

After the leaching procedure described above, primarily 0, 7, 14, 21 and 28 days of leaching, the uncoated polyurethane samples showed significant bacterial overgrowth or colonization with the organisms Escherichia coli and Staphylococcus aureus according to the described microbial test method.
**Example** 1 - A typical medical base formulation for the application of the curable antimicrobial coating composition of the present invention were prepared using the starting coating solution according to US Patent 4,642,267, Example 1, as follows:
   To a mixture of 75 g diacetone alcohol and 25 g methyl ethyl ketone is added 4 g polyvinylpyrrolidone (Kollidon 90, BASF Corp.) and 2 g linear polyurethane (Estane 5703, B. F. Goodrich Co.). To 10 g of the resulting solution was added 0.5g of a linear polyurethane polyisocyanate prepolymer (NORDOT Adhesive 34D-2, Synthetic Surfaces, Inc.) and 0.25g of the quaternary ammonium compound 3-chloro-2-hydroxypropyl-stearyl-dimethyl ammonium chloride (Quab 426). The resulting solution was applied to such substrates as polyurethane resins and permitted to dry. The resulting coating was a highly durable coating, which was slippery when wet and had antimicrobial property by preventing bacterial colonization without depletion of efficacy over extended period of leaching. No zone of inhibition was detectable after the initial burst and release of unreacted quat during initial leaching.
**Example 2** - A typical anti-fog base formulation for the application of the curable antimicrobial coating composition of the present invention were prepared using the starting coating solution according to US Patent 4,467,073, Example 1, as follows:
   2.5 g, Polyvinylpyrrolidone, PVP-K90, was dissolved in 100 ml of a mixture of 75% diacetone alcohol and 25% cyclohexane, followed by 1.0 g dioctyl sodium sulfosuccinate surfactant and 5.0 g Tycel 7351 isocyanate prepolymer (Hughson Chemicals, Lord Corporation). To 10 g of the resulting solution was added 0.5g of a linear polyurethane polyisocyanate prepolymer (NORDOT Adhesive 34D-2, Synthetic Surfaces, Inc.) and 0.25g of the quaternary ammonium compound 3-chloro-2-hydroxypropyl-cocoalkyl-dimethyl ammonium chloride (Quab 360). Coatings applied according to this composition and cured 24 hours at 22.2 °C (72 °F) were transparent, colorless, hard and scratch resistant and did not fog when cooled to 0 °C (32 °F) and then held over a beaker of boiling water. The coating had excellent adhesion to polycarbonate, polyester, polymethylmethacrylate and cellulose acetate plastics and had antimicrobial properties by preventing bacterial colonization without depletion of efficacy over extended period of leaching. No zone of inhibition was detectable after the initial burst and release of unreacted quat during initial leaching.
**Example 3** - **(Comparative Example)** A typical medical base formulation was prepared according to US Patent 4,642,267, Example 2, as follows:
   To 47 g of water and 10 g N-methylpyrrolidone is added 10 g of polyvinylpyrrolidone (Kollidon 90, BASF Corp.) and 33 g of linear polyurethane aqueous dispersion (Neorez R940, Polyvinyl Chemical Industries). Films cast from the resulting viscous dispersion were lubricious when wet (coefficient of friction 0.08) and imbibe water forming elastic, transparent films useful as burn and wound dressings. The solution can also be used to spin fibers which are tough and elastic when wet and can be used to produce hydrophilic foams via either mechanical frothing or casting films with added acetone and drying with heat in vacuum.
**Example 4** - To a mixture of 75 g diacetone alcohol and 25 g methyl ethyl ketone is added 4 g polyvinylpyrrolidone (Kollidon 90, BASF Corp.), 2 g linear polyurethane polyisocyanate prepolymer (NORDOT Adhesive 34D-2, Synthetic Surfaces, Inc.). To 10 g of the resulting solution was added 0.5g of a linear polyurethane polyisocyanate prepolymer (NORDOT Adhesive 34D-2, Synthetic Surfaces, Inc.) and 0.25g of alkyl hydroxyethyl dimethyl R ammonium chloride (R= C12) Preapagen HY (Clarient). The resulting solution was applied to a cleaned polyurethane slide by coating one side, air-dried and cured according to the sample preparation described above and leached in saline solution at room temperature for 0, 1, 7, 14, 21 and 28 days. Significant growth was observed on the sample after 7 days of leaching and all following weeks with St. aureus under the conditions of the described microbial test method, but no growth or colonization respectively was observed after all leaching periods and exposure to E. coli organisms. Thus, the above composition showed extensive efficacy against Escherichia coli, but failed after 7 days against Staphylococcus aureus.
**Example 5** - **(Comparative Example)** A typical medical base formulation containing no non-leaching antimicrobial according to US Patent 6,054,504, Example 3, was prepared as follows:
   Two grams of polyurethane polyisocyanate prepolymer (NORDOT Adhesive 34D-2, Synthetic Surfaces, Inc.) prepared by reaction of a 2 molar excess of diphenylmethane diisocyanate (MDI) with ricinoleate polyol, was combined with 35 g of methyl ethyl ketone, 10 g tetrahydrofuran, 10 g N-methylpyrrolidinone, 30 g diacetone alcohol, 3 g polyvinylpyrrolidinone (KOLLIDON 90F, BASF). A cleaned polyvinyl chloride slide was coated with the solution using a cotton swab. The slide was air-dried for 30 minutes and cured at 80 °C for 30 minutes.
   A polyurethane substrate instead of PVC was used and coated by dipping. The dip-coated sample was leached according to the sample preparation mentioned above and exposed to Escherichia coli organisms. In every case the samples showed significant bacterial overgrowth under the conditions of the described microbial test method.
**Example 6** - **(Comparative Example)** Another dip-coated sample was treated according to the sample preparation mentioned in Example 5 and exposed to Staphylococus aureus organisms after leaching the sample according to the method above. In every case the samples showed significant bacterial overgrowth under the conditions of the described microbial test method.
**Example 7** - To a mixture of 75 g diacetone alcohol and 25 g methyl ethyl ketone is added 4 g polyvinylpyrrolidone (Kollidon 90, BASF Corp.), 2 g linear polyurethane polyisocyanate prepolymer (NORDOT Adhesive 34D-2, Synthetic Surfaces, Inc.). To 10 g of the resulting solution was added 0.5g of a linear polyurethane polyisocyanate prepolymer (NORDOT Adhesive 34D-2, Synthetic Surfaces, Inc.) and 0.25g of 3-chloro-2-hydroxypropyl-lauryl dimethyl ammonium chloride, Quab 342 (Degussa). The resulting solution was applied to a cleaned polyurethane slide by coating one side, air-dried and cured according to the sample preparation described above and leached in saline solution at room temperature according to the method mentioned above. Growth or colonization respectively started to show on the sample after 7 days of leaching and all following weeks with St.aureus under the conditions of the described microbial test method. With the exposure to E. Coli the growth or colonization respectively started to show after 14 days of leaching.
**Example 8 - (Comparative Example)** Two grams of the polyurethane polyisocyanate prepolymer (NORDOT Adhesive 34D-2, Synthetic Surfaces, Inc.) prepared by reaction of a 2 molar excess of diphenylmethane diisocyanate (MDI) with ricinoleate polyol, was combined with 35 g of methyl ethyl ketone, 10 g tetrahydrofuran, 10 g N-methylpyrrolidinone, 30 g diacetone alcohol, 3 g polyvinylpyrrolidinone (KOLLIDON 90F, BASF). A cleaned polyurethane slide was coated with the solution on one side, air-dried and cured according to the sample preparation described above and leached in saline solution at room temperature according to the method mentioned above. After each time of leaching the samples showed significant bacterial overgrowth under the conditions of the described microbial test method.
**Example 9 - (Comparative Example)** To a mixture of 75 g diacetone alcohol and 25 g methyl ethyl ketone is added 4 g polyvinylpyrrolidone (Kollidon 90, BASF Corp.), 2 g linear polyurethane polyurethane polyisocyanate prepolymer (NORDOT Adhesive 34D-2, Synthetic Surfaces, Inc.). To 10 g of the resulting solution was added 0.5g of a linear polyurethane polyisocyanate prepolymer (NORDOT Adhesive 34D-2, Synthetic Surfaces, Inc.) and 0.25g of a siloxane modified quaternary ammonium compound 3-(trimethoxysilyl)propyldimethyloctadecyl ammonium chloride according to US patent 5,954,869. The resulting solution was applied to a cleaned polyurethane slide by coating one side, air-dried and cured according to the sample preparation described above and leached in saline solution at room temperature according to the method mentioned above. No Growth was observed after one day of leaching, but after 7 days of leaching and all following weeks the sample showed significant bacterial overgrowth with St. aureus under the conditions of the described microbial test method.
**Example 10 - (Comperative Example)** To a mixture of 75 g diacetone alcohol and 25 g methyl ethyl ketone is added 4 g polyvinylpyrrolidone (Kollidon 90, BASF Corp.), 2 g linear polyurethane polyurethane polyisocyanate prepolymer (NORDOT Adhesive 34D-2, Synthetic Surfaces, Inc.). To 10 g of the resulting solution was added 0.5g of a linear polyurethane polyurethane polyisocyanate prepolymer (NORDOT Adhesive 34D-2, Synthetic Surfaces, Inc.) and 0.25g of a siloxane modified quaternary ammonium compound 3-(trimethoxysilyl)propyldimethyloctadecyl ammonium chloride according to US patent 5,954,869. The resulting solution was applied to a cleaned polyurethane slide by coating one side, air-dried and cured according to the sample preparation described above and leached in saline solution at room temperature according to the method mentioned above. Significant growth was observed on the sample after one day of leaching and all following weeks with E. coli under the conditions of the described microbial test method.
**Example 11 - (Comparative Example)** To a mixture of 75 g diacetone alcohol and 25 g methyl ethyl ketone is added 4 g polyvinylpyrrolidone (Kollidon 90, BASF Corp.), 2 g linear polyurethane polyisocyanate prepolymer (NORDOT Adhesive 34D-2, Synthetic Surfaces, Inc.). To 10 g of the resulting solution was added 0.5g of a linear polyurethane polyisocyanate prepolymer (NORDOT Adhesive 34D-2, Synthetic Surfaces, Inc.) and 0.25g polyvinylpyrrolidone modified quaternary ammonium compound Styleze W-20 (ISP). Styleze W-20 is a PVP modified long chain quat that does not have a reactive group for covalent bonding according to the present invention. The resulting solution was applied to a cleaned polyurethane slide by coating one side, air-dried and cured according to the sample preparation described above and leached in saline solution at room temperature according to the method mentioned above. Significant growth was observed on the sample after one day of leaching and all following weeks with E. coli and St. under the conditions of the described microbial test method.
**Example 12 - (Comperative Example)** To a mixture of 75 g diacetone alcohol and 25 g methyl ethyl ketone is added 4 g polyvinylpyrrolidone (Kollidon 90, BASF Corp.), 2 g linear polyurethane polyurethane polyisocyanate prepolymer (NORDOT Adhesive 34D-2, Synthetic Surfaces, Inc.). To 10 g of the resulting solution was added 0.5g of a linear polyurethane polyisocyanate prepolymer (NORDOT Adhesive 34D-2, Synthetic Surfaces, Inc.) and 0.25g of di-oleic acid triethanolamine ester quat (Preapagen 4317) (Clarient). Preapagen 4317 is a di-oleic long chain acid tritethanol ester quat with no reactive group on the chain to form a covalent bond with the polymer matrix. The resulting solution was applied to a cleaned polyurethane slide by coating one side, air-dried and cured according to the sample preparation described above and leached in saline solution at room temperature according to the method mentioned above. Significant growth was observed on the sample after one day of leaching and all following weeks with E. coli and St. au. under the conditions of the described microbial test method.
**Example 13** - To a mixture of 75 g diacetone alcohol and 25 g methyl ethyl ketone is added 4 g polyvinylpyrrolidone (Kollidon 90, BASF Corp.), 2 g linear polyurethane polyisocyanate prepolymer (NORDOT Adhesive 34D-2, Synthetic Surfaces, Inc.). To 10 g of the resulting solution was added 0.5g of a linear polyurethane polyisocyanate prepolymer (NORDOT Adhesive 34D-2, Synthetic Surfaces, Inc.) and 0.25g of 3-chloro-2-hydroxypropyl-cocoalkyl dimethyl ammonium chloride, Quab 360 (Degussa). The resulting solution was applied to a cleaned polyurethane slide by coating one side, air-dried and cured according to the sample preparation described above and leached in saline solution at room temperature according to the method mentioned above. Growth or colonization respectively started to show on the sample after 7 days of leaching and all following weeks with St.aureus under the conditions of the described microbial test method. With the exposure to E. Coli the growth or colonization respectively started to show after 14 days of leaching.
**Example 14** - To a mixture of 75 g diacetone alcohol and 25 g methyl ethyl ketone is added 4 g polyvinylpyrrolidone (Kollidon 90, BASF Corp.), 2 g linear polyurethane polyurethane polyisocyanate prepolymer (NORDOT Adhesive 34D-2, Synthetic Surfaces, Inc.). To 10 g of the resulting solution was added 0.5g of a linear polyurethane polyisocyanate prepolymer (NORDOT Adhesive 34D-2, Synthetic Surfaces, Inc.) and 0.25g of 3-chloro-2-hydroxypropyl-stearyl dimethyl ammonium chloride, Quab 426 (Degussa). The resulting solution was applied to a cleaned polyurethane slide by coating one side, air-dried and cured according to the sample preparation described above and leached in saline solution at room temperature according to the method mentioned above. No growth or colonization respectively showed on the sample after all leaching periods with St.aureus under the conditions of the described microbial test method. With the exposure to E. coli the growth or colonization respectively started to show after 14 days of leaching.
**Example 15** - The antimicrobial coating was prepared by mixing 48.0% methyl ethyl ketone, 13.0% tetrahydrofuran, 12.0% ethyl lactate, 25.0% of a 12% PVP solution in ethyl lactate and 2 g linear polyurethane polyisocyanate prepolymer (NORDOT Adhesive 34D-2, Synthetic Surfaces, Inc.). To 10 g of the resulting solution was added 0.5g of a linear polyurethane polyisocyanate prepolymer (NORDOT Adhesive 34D-2, Synthetic Surfaces, Inc.) and 0.125g of 3-chloro-2-hydroxypropyl-stearyl dimethyl ammonium chloride, (Quab 426 from Degussa) and 0.125g of alkyl hydroxyethyl dimethyl -R- ammonium chloride (R= C12) (Preapagen HY from Clarient). The resulting solution was applied to a cleaned polyurethane slide by coating one side, air-dried and cured according to the sample preparation described above and leached in saline solution at room temperature according to the method mentioned above. No growth or colonization respectively was detected on the sample after all leaching periods up to 3 months with St.aureus and up to 6.5 months with E. coli individually tested under the conditions of the described microbial test.
**Example 16** - Example 15 was repeated with the same formulation and test sample preparation. Test organism tested was Streptococcus uberis. Leaching was in saline solution at room temperature according to the method mentioned above. No growth or colonization respectively was detected on the sample up to 56 days of leaching under the conditions of the described microbial test.
**Example 17** - **(Comparative Example from Patent** US 6,054,504**)** To a mixture of 5 grams of a linear polyurethane polyisocyanate prepolymer (NORDOT Adhesive 34D-2, Synthetic Surfaces, Inc), 48.26 grams of methyl ethyl ketone and 0.26 grams of Hexetidine (Clariant LSM) was added 13.56 grams of tetrahydrofuran, 12.68 grams of ethyl lactate and 23.57 grams of a 12% PVP K90 solution in ethyl lactate (2.82 grams polyvinylpyrrolidone). This solution was mixed and pipetted onto a polyurethane film, dried at room temperature for 10 minutes and cured in the oven between 60 and 70 °C for 45 minutes. These samples were then tested against bacterial growth of a gram-negative bacteria, Escherichia coli, and two gram-positive bacteria, Staphylococcus aureus and Staphylococcus epidermis. Films were tested after one day of leaching in phosphate buffer solution (PBS) at room temperature. The results showed rampant bacteria growth for all three types of bacteria. This leads to the conclusion that using hexetidine as a covalently bonded antibacterial component is unsuccessful. Further leaching of the coating is unnecessary due to failure after 24 hours.
**Example 18** - The formulation of Example 15 was tested over extended period of time in a second set-up but under the same leaching conditions as before. Escherichia coli, Staphylococcus aureus and Pseudomonas aeruginosa were used as test organisms. For over 3 months no colonization could be detected for all organisms on the treated surfaces whereas the controls showed growth.
**Example 19** - **(Comparative Example)** Stainless steel was prepared for testing an antimicrobial coating by applying an appropriate primer and cured for ten minutes at 80 °C. Then a second coat of a hydrophilic formulation cured for 12 hours at 80 °C was added on top of the primer. A third antimicrobial coating was coated on top of the two coatings that was prepared as follows: To a compound of 5 grams of a linear polyurethane polyisocyanate prepolymer (NORDOT Adhesive 34D-2, Synthetic Surfaces, Inc) was added 46.98 grams of methyl ethyl ketone, 13.20 grams of tetrahydrofuran, 12.34 grams ethyl lactate, 0.935 grams of Praepagen HY (Clariant), and 0.935 grams Quad 426 (Degussa). The stainless steel coating showed antimicrobial activity for at least two weeks.
**Example 20** - **(Comparative example with non-bonding quat)** An antimicrobial coating was prepared by mixing 48.0% methyl ethyl ketone, 13.0% tetrahydrofuran, 12.0% ethyl lactate, 25.0% ethyl lactate-PVP solution and 2 g linear polyurethane polyisocyanate prepolymer (NORDOT Adhesive 34D-2, Synthetic Surfaces, Inc.). To 10 g of the resulting solution was added 0.5g of a linear polyurethane polyisocyanate prepolymer (NORDOT Adhesive 34D-2, Synthetic Surfaces, Inc.) and 0.25g of benzalkonium chloride (CAS # 63449-41-2). The resulting solution was applied to a cleaned polyurethane slide by coating one side, air-dried and cured according to the sample preparation described above and leached in saline solution at room temperature according to the method mentioned above. After leaching for three days in phosphate buffer solution at room temperature, this coating solution shows limited efficacy against Staphylococcus aureus. By GC analysis it was found that after 3 days of leaching a concentration of only 1 to 2 ppm of benzalkonium chloride could be detected, whereas after leaching for one day 300-400 ppm and after leaching for 2 days 5-10 ppm was detectable. The detection level of day 2 coincides with the MIC level for this quat of about 7.5ppm. The coating showed efficacy against E. coli for up to about 3 weeks with slight colonization after that time. St. aureus showed no growth of up to three days and had significant surface growth thereafter.
**Example 21** - An antimicrobial coating was prepared by mixing 48.0% methyl ethyl ketone, 13.0% tetrahydrofuran, 12.0% ethyl lactate, 25.0% ethyl lactate-PVP solution and 2 g linear polyurethane polyisocyanate prepolymer (NORDOT Adhesive 34D-2, Synthetic Surfaces, Inc.). To 10 g of the resulting solution was added 0.5g of a linear polyurethane polyisocyanate prepolymer (NORDOT Adhesive 34D-2, Synthetic Surfaces, Inc.), 1.0% Praepagen HY (Clariant), and 1.0% Quab 426 (Degussa, CAS # 3001-63-6, CAS # 57-55-6, CAS # 7732-18-5). The resulting coating solution was applied to cleaned polyurethane sheets, air dried for 15 minutes at room temperature, cured at 80°C for one hour and allowed to react for an additional 24 hours at room temperature before any tests were performed. The coated polyurethane was then placed in an autoclave. The autoclave cycle conditions were 40 minutes at 121°C and 0.1 MPa (15 psi). This cycle was repeated six times. After each autoclave cycle, two pieces of polyurethane were cut from the coated and autoclaved sheet. The approximate size of the piece was 2.5 cm by 2.5 cm (one inch by one inch). One cut piece was used to test Escherichia coli and the other for Staphylococcus aureus. A 40µl sample of bacteria was pipetted onto the surface of the coated, autoclaved polyurethane. The inoculated polyurethane was left in an incubator at 37°C for 24 hours before viewing for growth. The coated samples still had efficacy against E. coli and S. aureus through 6 cycles of autoclaving as the method for sterilization.
**Example 22** - 10.8 grams of polyvinylpyrrolidone/dimethylacrylic acid (ISP) were added to 48 grams of water and thoroughly mixed, pH was adjusted with 0.1N HCl to about 5 and the mix heated and kept at 70 °C for 1 hr. 1.2 grams of the quat QUAB 426 was added, the mix stirred for 2 hrs and adjusted to pH 7 with a IN sodium hydroxide solution. 2.5% of this composition was incorporated together with 2.5% TWEEN 20 into a standard medical coating formulation according to example 2 of patent US patent 4,642,267 including a crosslinker. For making the standard medical coating, 47 g of water and 10 g N-methylpyrrolidone are added to 10 g of polyvinylpyrrolidone (Kollidon 90, BASF Corp.), 33g of linear polyurethane aqueous dispersion (Neorez R940, Polyvinyl Chemical Industries) and 0.1g aziridine (CX100). Samples were prepared by coating 2.5 cm x 5.0 cm (1"x2") polycarbonate pieces with the composition described above, cured at 100 °C for 1hr and tested for long term antimicrobial efficacy after leaching. The samples were leached in saline solution at room temperature according to the method mentioned above and exposed to the bacteria E. coli and St. aureus. No bacterial growth or bacterial colonization was detected after leaching for at least one week.
**Example 23** - A sample coated according to Example 15 was tested for its cytotoxicity potential by using Murine L929 fibroblast cells. The coated sample was soaked in media for 24hrs and then removed. Cells in that media survived whereas, in a control of a leaching biocide, the cells showed almost 100% necrosis.
**Example 24** - Polyurethane films were coated with the formula according to Example 15 and tested for anticoagulation. An uncoated sample and coated samples according to Example 3 were used as control. Fresh citrated human whole blood was reactivated by adding calcium chloride (0.02M). 50µl reactivated human blood was dropped on both coated and non-coated polyurethane facing up. The coated and uncoated polyurethane samples were put face up on a 10 cm slope with an angle of about 30 degrees. A drop of reactivated blood drop was put on each top part of the slope. On the non-coated control, as well as on the sample with a standard lubricious coating, the drop of blood did not move downwards but developed coagulation indicated by remaining at the spot where it was placed. The drop put on the antimicrobial sample coated according to the present invention moved downwards by gravity. It continuously ran down reaching the bottom of the sample within 10 minutes. The results show that the non-leaching antimicrobial polymeric coating composition according to the present invention when coated and cured on a polyurethane substrate does not cause coagulation on the coated substrate.

Thus, while there has been disclosed what is presently believed to be preferred embodiments of the invention, those skilled in the art will appreciate that other and further changes and modifications can be made without departing from the scope of the present invention as defined by the appended claims.

## Claims

1. A curable antimicrobial coating composition comprising:
(a) a polymeric matrix which comprises at least one polyurethane prepolymer, wherein said polyurethane prepolymer comprises a polyurethane backbone comprising at least one functional group capable of forming a chemical bond with the below-defined functional group of the long chain cationic surfactant, either directly or through a crosslinker, upon drying or curing of said coating composition;
(b) a carrier solvent;
(c) at least one long chain cationic surfactant compound comprising a functional group capable of forming a chemical bond with said polyurethane prepolymer upon evaporating said carrier solvent and drying or curing of said composition, said functional group being selected from the group consisting of an amine, thiol, carboxyl, aldehyde, hydroxyl and combinations thereof; and
(d) at least one hydrophilic organic monomer, oligomer, prepolymer, polymer or copolymer derived from vinyl alcohol, N-vinylpyrrolidone, N-vinyl lactam, acrylamide, amide, styrenesulfonic acid, combination of vinylbutyral and N-vinylpyrrolidone, hydroxyethyl methacrylate, acrylic acid, vinylmethyl ether, vinylpyridylium halide, methyl cellulose, ethyl cellulose, carboxymethyl cellulose, hydroxyethyl cellulose, hydroxypropyl cellulose, hydroxymethyl ethyl cellulose, hydroxypropylmethyl cellulose, cellulose acetate, cellulose nitrate, starch, gelatin, albumin, casein, gum, alginate, hydroxyethyl (meth)acrylate, hydroxypropyl (meth)acrylate, ethylene glycol (meth)acrylates (e.g. triethylene glycol (meth)acrylate) and meth)acrylamide), N-alkyl (meth) acrylamides (e.g. N-methyl (meth)acrylamide and N-hexyl (meth)acrylamide), N,N-dialkyl (meth)acrylamides (e.g. N,N-dimethyl (meth)acrylamide and poly-N,N-dipropyl (meth)acrylamide), N-hydroxyalkyl (meth)acrylamide polymers, such as poly-N-methylol (meth)acrylamide and poly-N-hydroxy ethyl (meth)acrylamide, and N,N-dihydroxyalkyl (meth)acrylamide polymers, such as poly-N,N-dihydroxyethyl (meth)acrylamide, ether polyols, polyethylene oxide, polypropylene oxide, and poly(vinyl ether), alkylvinyl sulfones, alkylvinylsulfone-acrylates or a combination thereof;
wherein said at least one long chain cationic surfactant compound is non-leaching upon drying or curing of said coating composition and projects at least 15Å away from the surface of the cured coating composition to thereby protrude through and beyond organic debris deposited over time on the surface of said cured composition, wherein said organic debris are dead microbial cells, proteinaceous buildup or a combination thereof.

2. A curable antimicrobial coating composition according to claim 1, wherein said at least one hydrophilic organic monomer, oligomer, prepolymer, polymer or copolymer is present in an amount whereby a surface coated with the cured coating composition has at least 70% less friction when compared to the uncoated surface when each are wetted with water or an aqueous solution.

3. A curable antimicrobial coating composition according to any of the previous claims, wherein said cationic surfactant is a quaternary ammonium compound.

4. A curable antimicrobial coating composition according to claim 3, wherein said quaternary ammonium compound is selected from the group consisting of an alkyl hydroxyethyl dimethyl ammonium chloride; polyquaternium 11; a quaternized copolymer of vinylpyrrolidone and dimethylaminoethylmethacrylate; polyquaternium 16; polyquaternium 44; a combination of a vinylpyrrolidone and quaternized vinylimidazol; polyquaternium-55; a quaternized copolymer of vinylpyrrolidone and dimethylaminoethyl; N,N-dimethyl-N-dodecyl-N-(2-hydroxy- 3-sulfopropyl) ammonium betaine; N-alkyl acid amidopropyl-N,N-dimethyl-N-(3- sulfopropyl)-ammonium betaine; 3-chloro-2-hydroxypropyl-alkyl-dimethylammonium chloride with a long chain alkyl group; and combinations thereof.

5. A curable antimicrobial coating composition according to any of the previous claims, wherein said surfactant projects at least 30 Å away from the surface of said cured coating composition, or at least 60 Å away from the surface of said cured coating composition.

6. A curable antimicrobial coating composition according to claim 1 comprising:
(a) from 0.01% to 20% of said at least one polyurethane prepolymer based on the weight of the composition;
(b) at least one carrier solvent capable of at least partially dissolving said polyurethane prepolymer, present in an amount from 99.89% to 75% based on the weight of the composition;
(c) a hydrophilic component as defined in claim 1, present in an amount from 0.01 to 40 % based on the weight of the composition; and
(d) at least one quaternary ammonium compound present in an amount from 0.01% to 5% based on the weight of the composition and selected from the group consisting of an alkyl hydroxyethyl dimethyl ammonium chloride; polyquaternium 11; a quaternized copolymer of vinylpyrrolidone and dimethylaminoethylmethacrylate; polyquaternium 16; polyquaternium 44; a combination of a vinylpyrrolidone and quaternized vinylimidazol; polyquaternium-55; a quaternized copolymer of vinylpyrrolidone and dimethylaminoethyl; N,N-dimethyl-N-dodecyl-N-(2-hydroxy- 3-sulfopropyl) ammonium betaine; N-alkyl acid amidopropyl-N,N-dimethyl-N-(3- sulfopropyl)-ammonium betaine; 3-chloro-2-hydroxypropyl-alkyl-dimethylammonium chloride with a long chain alkyl group; and combinations thereof.

7. A curable antimicrobial coating composition according to claim 6, further comprising a modifying polymer selected from the group consisting of polyester, polyalkyl, maleic anhydride polymer, maleic anhydride copolymer, polyol, polyamine, polyacrylate, polyvinyl acetate, polyglucosamid, polyglucosamine, their copolymers and combinations thereof.

8. A curable antimicrobial coating composition according to claim 6, wherein said hydrophilic polymer, copolymer or prepolymer is present in an amount from 0.2% to 15% based on the weight of the composition in replacement of said carrier solvent.

9. A curable antimicrobial coating composition according to claim 8, wherein said hydrophilic polymer, copolymer or prepolymer is N-polyvinylpyrrolidone.

10. A curable antimicrobial coating composition according to claim 6, further comprising a crosslinker selected from the group consisting of an aziridine, carbdiimide, melamine, multifunctional alcohol, multifunctional aldehyde, multifunctional amine, multifunctional isocyanate and combinations thereof.

11. A curable antimicrobial coating composition according to claim 10, wherein said crosslinker is present in an amount from 0.001% to 5% based on the weight of the composition in replacement of said carrier solvent.

12. A curable antimicrobial coating composition according to claim 6, further comprising a reaction enhancing catalyst.

13. A curable antimicrobial coating composition according to claim 12, wherein said catalyst is selected from the group consisting of tin organic compounds, cobalt organic compounds, triethylamine and combinations thereof.

14. A curable antimicrobial coating composition according to claim 6, wherein said carrier solvent is selected from the group consisting of water, methyl ethyl ketone, N-methylpyrrolidone, tetrahydrofuran, dichloromethane, chloroform, ethyl acetate, propylene glycol methyl ether, propylene glycol methyl ether acetate, diacetone alcohol, ether, ester, aromatic hydrocarbon, chlorinated hydrocarbon, linear hydrocarbon and combinations thereof.

15. A curable antimicrobial coating composition according to claim 6, further comprising an additional component intended to leach out of the cured coating composition or to be bonded with a crosslinker, which additional component is selected from the group consisting of an antimicrobial compound, biocide, antibiotic, drug, vitamin, fungicide, fungistat, virucide, germicide, spermacide, therapeutic agent, heparin, plant extract and combinations thereof.

## Patentansprüche

1. Härtbare antimikrobielle Beschichtungszusammensetzung umfassend:
(a) Eine polymerische Matrix, welche mindestens ein Polyurethan-Prepolymer umfasst, wobei das Polyurethan-Prepolymer ein Polyurethan Grundgerüst umfasst, umfassend mindestens eine funktionelle Gruppe geeignet zum Bilden einer chemischen Bindung mit der unten-definierten funktionellen Gruppe von dem lang-kettigen kationischen Tensid, entweder direkt oder durch ein Vernetzungsmittel, nach Trocknen oder Härten von der Beschichtungszusammensetzung;
(b) ein Trägerlösungsmittel;
(c) mindestens eine lang-kettige kationische Tensid-Verbindung umfassend eine funktionelle Gruppe geeignet zum Bilden von einer chemischen Bindung mit dem Polyurethan-Prepolymer nach Verdunsten des Trägerlösungsmittels und Trocknen oder Härten der Zusammensetzung, die funktionelle Gruppe ausgewählt aus der Gruppe bestehend aus einem Amin, Thiol, Carboxyl, Aldehyd, Hydroxyl und Kombinationen davon; und
(d) mindestens ein hydrophiles organisches Monomer, Oligomer, Prepolymer, Polymer oder Copolymer abgeleitet von Vinylalkohol, N-Vinylpyrrolidon, N-Vinyl-Lactam, Acrylamid, Amid, Styrolsulfonsäure, Kombination von Vinylbutyral und N-Vinylpyrrolidon, Hydroxyethyl-Methacrylat, Acrylsäure, Vinylmethyl-Ether, Vinylpyridylium-Halid, Methyl-Cellulose, Ethyl-Cellulose, Carboxymethyl-Cellulose, HydroxyethylCellulose, Hydroxypropyl-Cellulose, Hydroxymethyl-Ethyl-Cellulose, Hydroxypropylmethyl-Cellulose, Cellulose-Acetat, Cellulose-Nitrat, Stärke, Gelatine, Albumin, Casein, Gummi, Alginat, Hydroxyethyl-(Meth)acrylat, Hydroxypropyl-(Meth)acrylat, Ethylen-Glycol-(Meth)acrylaten (z.B. TriethylenGlycol-(Meth)acrylat und (Meth)acrylamid), N-Alkyl-(Meth)acrylamide (z.B. N-Methyl-(Meth)acrylamid und N-Hexyl-(Meth)acrylamid), N,N-dialkyl-(Meth)acrylamide (z.B. N,N-dimethyl-(Meth)acrylamid und Poly-N,N-Dipropyl-(meth)acrylamid), N-Hydroxyalkyl-(Meth)acrylamid-Polymere, wie Poly-N-Methylol-(Meth)acrylamid und Poly-N-HydroxyEthyl-(Meth)acrylamid, und N,N-Dihydroxyalkyl-(Meth)acrylamid-Polymere, wie Poly-N,N-Dihydroxyethyl-(Meth)acrylamid, Ether-Polyole, Polyethylen-Oxid, Polypropylen-Oxid, und Poly(Vinyl-Ether), Alkylvinyl-Sulfone, Alkylvinylsulfon-Acrylate oder eine Kombination davon; wobei die mindestens eine lang-kettige kationische Tensid-Verbindung nicht-auslaugend ist nach Trocknen oder Härten von der Beschichtungszusammensetzung und mindestens 15Ä weg von der Oberfläche von der gehärteten Beschichtungszusammensetzung ragt, um dabei durch und über organische Ablagerungen, abgesetzt über die Zeit auf der Oberfläche von der gehärteten Zusammensetzung vorzustehen, wobei die organischen Ablagerungen tote mikrobielle Zellen, proteinhaltige Anhäufungen oder eine Kombination davon sind.

2. Härtbare antimikrobielle Beschichtungszusammensetzung nach Anspruch 1, wobei das mindestens eine hydrophile organische Monomer, Oligomer, Prepolymer, Polymer oder Copolymer in einer Menge vorhanden ist, wodurch eine Oberfläche beschichtet mit der gehärteten Beschichtungszusammensetzung mindestens 70% weniger Reibung hat, wenn verglichen mit der unbeschichteten Oberfläche, wenn jede mit Wasser oder einer wässrigen Lösung befeuchtet ist.

3. Härtbare antimikrobielle Beschichtungszusammensetzung nach einem der vorhergehenden Ansprüche, wobei das kationische Tensid eine quaternäre Ammonium-Verbindung ist.

4. Härtbare antimikrobielle Beschichtungszusammensetzung nach Anspruch 3, wobei die quaternäre Ammonium-Verbindung ausgewählt ist aus der Gruppe bestehend aus einem Alkyl-Hydroxyethyl-Dimethyl-Ammonium-Chlorid; Polyquaternium 11; einem quaternisierten Copolymer von Vinylpyrrolidon und Diemethylaminoethylmethacrylat; Polyquaternium 16; Polyquaternium 44; eine Kombination von einem Vinylpyrrolidon und quaternisiertem Vinylimidazol; Polyquaternium-55; einem quaternisierten Copolymer von Vinylpyrrolidon und Dimethylaminoethyl; N,N-Dimethyl-N-Dodecyl-N-(2-Hydroxy-3-Sulfopropyl)-Ammonium-Betaine; N-Alkylsäure-Amidopropyl-N,N-Dimethyl-N-(3-Sulfopropyl)-Ammonium-Betaine; 3-Chloro-2-Hydroxypropyl-Alkyldimethylammonium-Chlorid mit einer lang-kettigen Alkylgruppe; und Kombinationen davon.

5. Härtbare antimikrobielle Beschichtungszusammensetzung nach einem der vorhergehenden Ansprüche, wobei das Tensid mindestens 30 Å weg von der Oberfläche der gehärteten Beschichtungszusammensetzung ragt, oder mindestens 60 Å weg von der Oberfläche von der gehärteten Beschichtungszusammensetzung.

6. Härtbare antimikrobielle Beschichtungszusammensetzung nach Anspruch 1 umfassend:
(a) von 0,01% bis 20% von dem mindestens einen Polyurethan-Prepolymer, basierend auf dem Gewicht der Zusammensetzung;
(b) mindestens ein Trägerlösungsmittel geeignet zum mindestens partiellen Auflösen des Polyurethan-Prepolymers, vorhanden in einer Menge von 99,89% bis 75%, basierend auf dem Gewicht der Zusammensetzung;
(c) hydrophile Komponente wie in Anspruch 1 definiert, vorhanden in einer Menge von 0,01 bis 40 %, basierend auf dem Gewicht der Zusammensetzung; und
(d) mindestens eine quaternäre Ammonium-Verbindung, anwesend in einer Menge von 0,01% bis 5%, basierend auf dem Gewicht der Zusammensetzung und ausgewählt aus der Gruppe bestehend aus einem Alkyl-Hydroxyethyl-Dimethyl-Ammonium-Chlorid; Polyquaternium 11; einem quaternisierten Copolymer von Vinylpyrrolidon und Dimethylaminoethylmethacrylat; Polyquaternium 16; Polyquaternium 44; eine Kombination von einem Vinylpyrrolidon und quaternisiertem Vinylimidazol; Polyquaternium-55; einem quaternisierten Copolymer von Vinylpyrrolidon und Dimethylaminoethyl; N,N-dimethyl-N-dodecyl-N-(2-Hydroxy-3-Sulfopropyl)-Ammonium-Betaine; N-Alkylsäure-Amidopropyl-N,N-Dimethyl-N-(3-Sulfopropyl)-Ammonium-Betaine; 3-Chlor-2-Hydroxypropyl-Alkyl-Dimethylammonium-Chlorid mit einer lang-kettigen Alkylgruppe; und Kombinationen davon.

7. Härtbare antimikrobielle Beschichtungszusammensetzung nach Anspruch 6, weiter umfassend ein modifizierendes Polymer, ausgewählt von der Gruppe bestehend aus Polyester, Polyalkyl, Malein-Anhydrid-Polymer, Malein-Anhydrid-Copolymer, Polyol, Polyamin, Polyacrylat, Polyvinyl-Acetat, Polyglucosamid, Polyglucosamine, deren Copolymere und Kombinationen davon.

8. Härtbare antimikrobielle Beschichtungszusammensetzung nach Anspruch 6, wobei das hydrophile Polymer, Copolymer oder Prepolymer in einer Menge von 0,2% bis 15%, basierend auf dem Gewicht der Zusammensetzung im Austausch mit dem Träger-Lösungsmittel, vorhanden ist.

9. Härtbare antimikrobielle Beschichtungszusammensetzung nach Anspruch 8, wobei das hydrophile Polymer, Copolymer oder Prepolymer N-Polyvinylpyrrolidon ist.

10. Härtbare antimikrobielle Beschichtungszusammensetzung nach Anspruch 6, weiter umfassend ein Vernetzungsmittel ausgewählt aus der Gruppe bestehend aus einem Aziridin, Carbdiimid, Melamin, multifunktionalem Alkohol, multifunktionalem Aldehyd, multifunktionalem Amin, multifunktionalem Isocyanat und Kombinationen davon.

11. Härtbare antimikrobielle Beschichtungszusammensetzung nach Anspruch 10, wobei das Vernetzungsmittel in einer Menge von 0,001% bis 5%, basierend auf dem Gewicht der Zusammensetzung im Austausch mit dem Träger-Lösungsmittel vorhanden ist.

12. Härtbare antimikrobielle Beschichtungszusammensetzung nach Anspruch 6, weiter umfassend einen Reaktion-verbessernden Katalysator.

13. Härtbare antimikrobielle Beschichtungszusammensetzung nach Anspruch 12, wobei der Katalysator ausgewählt ist aus der Gruppe bestehend aus Zinn-organischen Verbindungen, Kobaltorganischen Verbindungen, Triethylamin und Kombinationen davon.

14. Härtbare antimikrobielle Beschichtungszusammensetzung nach Anspruch 6, wobei das Trägerlösungsmittel ausgewählt ist aus der Gruppe bestehend aus Wasser, Methyl-Ethyl-Keton, N-Methylpyrrolidone, Tetrahydrofuran, Dichlormethan, Chloroform, Ethyl-Acetat, Propylen-Glycol-Methyl-Ether, Propylen-Glycol-Methyl-Ether-Acetat, Diaceton-Alkohol, Ether, Ester, aromatisches Hydrocarbon, chloriertes Hydrocarbon, lineares Hydrocarbon und Kombinationen davon.

15. Härtbare antimikrobielle Beschichtungszusammensetzung nach Anspruch 6, weiter umfassend eine zusätzliche Komponente vorgesehen, um aus der gehärteten Beschichtungszusammensetzung auszulaugen, oder, um mit einem Vernetzungsmittel gebunden zu sein, wobei die zusätzliche Komponente ausgewählt ist aus der Gruppe bestehend aus einer antimikrobiellen Verbindung, Biozid, Antibiotikum, Medikament, Vitamin, Fungizid, Fungistatikum, Virizid, Germizid, Spermazid, therapeutischem Mittel, Heparin, Pflanzenextrakt und Kombinationen davon.

## Revendications

1. Composition de revêtement antimicrobienne durcissable comprenant :
(a) une matrice polymère qui comprend au moins un prépolymère de polyuréthanne, ledit prépolymère de polyuréthanne comprenant un squelette en polyuréthanne comprenant au moins un groupe fonctionnel capable de former une liaison chimique avec le groupe fonctionnel défini ci-dessous du tensioactif cationique à longue chaîne, directement ou par l'intermédiaire d'un agent de réticulation, lors du séchage ou du durcissement de ladite composition de revêtement ;
(b) un solvant de support ;
(c) au moins un composé tensioactif cationique à longue chaîne comprenant un groupe fonctionnel capable de former une liaison chimique avec ledit prépolymère de polyuréthane lors de l'évaporation dudit solvant de support et du séchage ou du durcissement de ladite composition, ledit groupe fonctionnel étant choisi parmi le groupe constitué d'une amine, thiol, carboxyle, aldéhyde, hydroxyle et de combinaisons de ceux-ci ; et
(d) au moins un monomère, oligomère, prépolymère, polymère ou copolymère organique hydrophile dérivé d'alcool vinylique, de N-vinylpyrrolidone, de N-vinyle lactame, d'acrylamide, d'amide, d'acide styrènesulfonique, d'une combinaison de vinylbutyral et de N-vinylpyrrolidone, de méthacrylate d'hydroxyéthyle, d'acide acrylique, d'éther de vinylméthyle, d'halogénure de vinylpyridylium, de méthylcellulose, d'éthylcellulose, de carboxyméthylcellulose, d'hydroxyéthylcellulose, d'hydroxypropylcellulose, d'hydroxyméthyléthylcellulose, d'hydroxypropylméthylcellulose, d'acétate de cellulose, de nitrate de cellulose, d'amidon, de gélatine, d'albumine, de caséine, de gomme, d'alginate, de (méth)acrylate d'hydroxyéthyle, de (méth)acrylate d'hydroxypropyle, de (méth)acrylates d'éthylèneglycol (par exemple (méth)acrylate de triéthylèneglycol et (méth)acrylamide), de N-alkyl(méth)acrylamides (par exemple N-méthyl(méth)acrylamide et N-hexyl(méth)acrylamide), de N,N-dialkyl(méth)acrylamides (par exemple, N,N-diméthyl(méth)acrylamide et poly-N,N-dipropyl(méth)acrylamide), de polymères de N-hydroxyalkyl(méth)acrylamide, tels que poly-N-méthylol(méth)acrylamide et poly-N-hydroxyéthyl(méth)acrylamide, et de polymères de N,N-dihydroxyalkyl(méth)acrylamide, tels que poly-N,N-dihydroxyéthyl(méth)acrylamide, d'éther polyols, d'oxyde de polyéthylène, d'oxyde de polypropylène, et de poly(vinyléther), d'alcylvinylsulfones, d'alcylvinylsulfone-acrylates ou d'une combinaison de ceux-ci ;
dans laquelle ledit au moins un composé tensioactif cationique à longue chaîne est non lixiviant lors du séchage ou du durcissement de ladite composition de revêtement et fait saillie sur au moins 15 Å depuis la surface de la composition de revêtement durcie pour ainsi faire saillie à travers et au-delà de débris organiques déposés dans le temps sur la surface de ladite composition durcie, dans laquelle lesdits débris organiques sont des cellules microbiennes mortes, une accumulation protéinique ou une combinaison de celles-ci.

2. Composition de revêtement antimicrobienne durcissable selon la revendication 1, dans laquelle ledit au moins un monomère, oligomère, prépolymère, polymère ou copolymère organique hydrophile est présent selon une quantité telle qu'une surface revêtue de la composition de revêtement durcie présente au moins 70 % de frottement en moins par rapport à la surface non revêtue lorsqu'elles sont chacune mouillées avec de l'eau ou une solution aqueuse.

3. Composition de revêtement antimicrobienne durcissable selon l'une quelconque des revendications précédentes, dans laquelle ledit tensioactif cationique est un composé d'ammonium quaternaire.

4. Composition de revêtement antimicrobienne durcissable selon la revendication 3, dans laquelle ledit composé d'ammonium quaternaire est choisi parmi le groupe constitué d'un chlorure d'alkylhydroxyéthyldiméthylammonium ; de polyquaternium 11 ; d'un copolymère quaternisé de vinylpyrrolidone et de diméthylaminoéthylméthacrylate ; de polyquaternium 16 ; de polyquaternium 44 ; d'une combinaison de vinylpyrrolidone et de vinylimidazol quaternisé ; de polyquaternium-55 ; d'un copolymère quaternisé de vinylpyrrolidone et de diméthylaminoéthyle ; de N,N-diméthyl-N-dodécyl-N-(2-hydroxy-3-sulfopropyl)ammonium bétaïne ; d'acide N-alkyle amidopropyl-N,N-diméthyl-N-(3-sulfopropyl)-ammonium bétaïne ; de chlorure de 3-chloro-2-hydroxypropyl-alkyl-diméthylammonium avec un groupe alkyle à longue chaîne ; et de combinaisons de ceux-ci.

5. Composition de revêtement antimicrobienne durcissable selon l'une quelconque des revendications précédentes, dans laquelle ledit tensioactif fait saillie sur au moins 30 Å depuis la surface de ladite composition de revêtement durcie, ou sur au moins 60 Å depuis la surface de ladite composition de revêtement durcie.

6. Composition de revêtement antimicrobienne durcissable selon la revendication 1, comprenant :
(a) de 0,01 % à 20 % dudit au moins un prépolymère de polyuréthane sur la base du poids de la composition ;
(b) au moins un solvant de support capable de dissoudre au moins partiellement ledit prépolymère de polyuréthane, présent selon une quantité de 99,89 % à 75 % sur la base du poids de la composition ;
(c) un composant hydrophile tel que défini selon la revendication 1, présent selon une quantité de 0,01 à 40 % sur la base du poids de la composition ; et
(d) au moins un composé d'ammonium quaternaire présent selon une quantité de 0,01 % à 5 % sur la base du poids de la composition et choisi parmi le groupe constitué d'un chlorure d'alkylhydroxyéthyldiméthylammonium ; de polyquaternium 11 ; d'un copolymère quaternisé de vinylpyrrolidone et de diméthylaminoéthylméthacrylate ; de polyquaternium 16 ; de polyquaternium 44 ; d'une combinaison de vinylpyrrolidone et de vinylimidazol quaternisé ; de polyquaternium-55 ; d'un copolymère quaternisé de vinylpyrrolidone et de diméthylaminoéthyle ; de N,N-diméthyl-N-dodécyl-N-(2-hydroxy-3-sulfopropyl)ammonium bétaïne ; d'acide N-alkyle amidopropyl-N,N-diméthyl-N-(3-sulfopropyl)-ammonium bétaïne ; de chlorure de 3-chloro-2-hydroxypropyl-alkyl-diméthylammonium avec un groupe alkyle à longue chaîne ; et de combinaisons de ceux-ci.

7. Composition de revêtement antimicrobienne durcissable selon la revendication 6, comprenant en outre un polymère modifiant choisi parmi le groupe constitué d'un polyester, polyalkyle, polymère d'anhydride maléique, copolymère d'anhydride maléique, polyol, polyamine, polyacrylate, acétate de polyvinyle, polyglucosamide, polyglucosamine, des copolymères et combinaisons de ceux-ci.

8. Composition de revêtement antimicrobienne durcissable selon la revendication 6, dans laquelle ledit polymère, copolymère ou prépolymère hydrophile est présent selon une quantité de 0,2 % à 15 % sur la base du poids de la composition en remplacement dudit solvant de support.

9. Composition de revêtement antimicrobienne durcissable selon la revendication 8, dans laquelle ledit polymère, copolymère ou prépolymère hydrophile est la N-polyvinylpyrrolidone.

10. Composition de revêtement antimicrobienne durcissable selon la revendication 6, comprenant en outre un agent de réticulation choisi parmi le groupe comprenant une aziridine, carbdiimide, mélamine, alcool multifonctionnel, aldéhyde multifonctionnel, amine multifonctionnelle, isocyanate multifonctionnel et des combinaisons de ceux-ci.

11. Composition de revêtement antimicrobienne durcissable selon la revendication 10, dans laquelle ledit agent de réticulation est présent selon une quantité de 0,001 % à 5 % sur la base du poids de la composition en remplacement dudit solvant de support.

12. Composition de revêtement antimicrobienne durcissable selon la revendication 6, comprenant en outre un catalyseur d'amélioration de réaction.

13. Composition de revêtement antimicrobienne durcissable selon la revendication 12, dans laquelle ledit catalyseur est choisi parmi le groupe constitué de composés organiques d'étain, composés organiques de cobalt, triéthylamine et de combinaisons de ceux-ci.

14. Composition de revêtement antimicrobienne durcissable selon la revendication 6, dans laquelle ledit solvant de support est choisi parmi le groupe constitué d'eau, méthyléthylcétone, N-méthylpyrrolidone, tétrahydrofurane, dichlorométhane, chloroforme, acétate d'éthyle, méthyléther de propylèneglycol,, méthyléther acétate de propylèneglycol, alcool diacétone, éther, ester, hydrocarbure aromatique, hydrocarbure chloré, hydrocarbure linéaire et de combinaisons de ceux-ci.

15. Composition de revêtement antimicrobienne durcissable selon la revendication 6, comprenant en outre un composant supplémentaire destiné à être éliminé par lixiviation de la composition de revêtement durcie ou à être liée à un agent de réticulation, lequel composant supplémentaire est choisi parmi le groupe comprenant un composé antimicrobien, biocide, antibiotique, médicament, vitamine, fongicide, fongistatique, virucide, germicide, spermicide, agent thérapeutique, héparine, extrait de plante et des combinaisons de ceux-ci.
